# FASCICULE DE BREVET EUROPEEN

(11) **EP 2 665 458 B1**
(45) Date de publication et mention de la délivrance du brevet: **07.01.2015**
(21) Numéro de dépôt: 12700845.6
(22) Date de dépôt: 23.01.2012
(51) Int. Cl.: A61J 1/03, A61J 7/00, G07F 11/62, G06F 19/00, B65D 75/32

(54) **SYSTEME AUTOMATISE DE DOSE UNITAIRE POUR LA DELIVRANCE NOMINATIVE DE MEDICAMENTS**
AUTOMATISIERTES DOSISSYSTEM ZUR PORTIONIERTEN ABGABE VON MEDIKAMENTEN
AUTOMATED UNIT-DOSE SYSTEM FOR THE UNIT-DOSE DISPENSING OF DRUGS

(30) Priorité: 21.01.2011 FR 1150504
(43) Date de publication de la demande: 27.11.2013
(73) Titulaire: Ethilog Sas, 59120 Loos (FR)
(72) Inventeur: SHAW, Henry, F-24000 Perigueux (FR); DEWULF, Stefaan, B-9850 Nevele (BE)
(74) Mandataire: Lebkiri, Alexandre
(86) Numéro de dépôt international: PCT/EP2012/050915
(87) Numéro de publication internationale: WO 2012/098249

(56) Documents cités:
- EP-A1- 1 176 106
- WO-A1-2010/095102

## Description

La présente invention se rapporte au domaine de la délivrance nominative de médicaments par exemple dans une institution collective, telle qu'hôpital, clinique, maison de repos ou prison, et faisant appel à une pharmacie centrale. La présente invention vise à procurer un système automatisé entièrement sécurisé pour la délivrance nominative de doses unitaires de médicaments aux patients, permettant de s'affranchir des coûts du personnel soignant utilisé actuellement pour la préparation manuelle de ces doses unitaires individualisées. La présente invention vise à procurer un système permettant une excellente traçabilité des doses unitaires de médicaments jusqu'à leur délivrance aux patients, de façon à éviter les erreurs de délivrance et de façon, en cas de problème de tolérance d'un patient à un médicament, à pouvoir identifier avec certitude le lot de fabrication du médicament ayant causé problème. La présente invention vise aussi à procurer un système permettant, le cas échéant et si nécessaire, d'établir une relation entre l'informatisation de la prescription et l'automatisation de la délivrance de cette prescription dans l'institution collective en question.

### ARRIERE-PLAN TECHNIQUE DE L'INVENTION

De nos jours les systèmes de préparation à la délivrance nominative de doses unitaires de médicaments aux patients présents dans une institution collective faisant appel à une pharmacie centrale tendent vers l'automatisation, de façon à réduire le coût de la préparation et la délivrance manuelle par le personnel soignant de l'institution, et vers une sécurisation accrue de manière à réduire fortement voire éviter les risques d'erreurs inévitablement associés à une préparation comprenant une plusieurs phases manuelles.

Un système de préparation à une délivrance automatisée et sécurisée doit pouvoir présenter plusieurs avantages :
1. amélioration des conditions d'hygiène en évitant les nombreux contacts de l'homme avec le médicament même,
2. respect de certaines réglementations nationales,
3. diminution du risque de contamination,
4. sécurisation du circuit du médicament à l'intérieur de l'institution (en particulier pour des médicaments à législation restrictive tels que des stupéfiants), et amélioration de la fiabilité,
5. gain de temps considérable pour le personnel soignant,
6. meilleure traçabilité des médicaments délivrés, par exemple par numéro de lot, date de péremption, composition, etc.,
7. meilleure gestion des stocks de médicaments,
8. limitation des pertes de médicaments par intégration des retours dans le circuit de délivrance,
9. amélioration de la réactivité de la logistique du circuit médicamenteux, par diminution des besoins d'en-cours,
10. réduction ou élimination d'entreposage difficilement sécurisé de médicaments préparés longtemps avant leur administration, et
11. transparence du circuit médicamenteux, permettant un réapprovisionnement en temps utile et une facturation ou une affectation des coûts correctes.

Il existe déjà dans la littérature des systèmes automatisés et sécurisés de délivrance nominative de doses unitaires de médicaments traçables, en particulier pour des médicaments conditionnés dans des plaquettes (blisters) à alvéoles.

Par exemple on peut citer le brevet américain n° 3.759.597 décrivant un système de dose unitaire qui minimise la quantité d'échange d'information à accomplir par plus d'une personne, ce système employant des supports de dose individuelle tels que des cartes informatives pouvant être traitées par une machine, ces cartes faisant partie de la dose unitaire avec toute l'information pharmaceutique pertinente (par exemple le nom commercial du médicament, le nom du principe actif, le dosage en principe actif, les effets secondaires, les incompatibilités, la stabilité, et le numéro de catalogue tel que le code de médicament national à neuf chiffres en vigueur aux Etats-Unis) et avec les indices d'identification pharmaceutique requis. Dans le cas où la carte doit être appropriée pour une opération de perforation, la dose unitaire est conditionnée dans une enveloppe substantiellement plate. Selon un mode de réalisation, le support comporte une dose individuelle qui lui est attachée de manière adhésive au moyen d'un adhésif ou film sur-enveloppant constitué d'une substance médicalement inerte. Le brevet américain n° 3.759.597 décrit aussi un système de stockage de ces ensembles dose-support, capable de récupérer un ensemble dose-support sélectionné de manière individuelle.

Le brevet européen n° 1.176.106 décrit un procédé pour préparer automatiquement les prises de médicaments d'un patient à partir d'une ordonnance informatisée prescrivant des médicaments déterminés pendant une période définie en un nombre déterminé de prises, les médicaments étant emballés dans leur conditionnement primaire d'origine se présentant sous la forme de plaquettes rassemblant dans des alvéoles respectives un nombre prédéterminé de doses unitaires d'un même médicament, caractérisé en ce qu'il comprend une phase préalable de pré-conditionnement des médicaments dans leur conditionnement primaire d'origine, cette phase de pré-conditionnement comprenant les étapes suivantes pour chaque médicament à stocker :
- l'identification de la référence du médicament,
- l'introduction et la mémorisation des emplacements respectifs sur la plaquette des doses unitaires du médicament,
- le pré-conditionnement des plaquettes en bandes,
- le rangement des bandes supportant le médicament dans un poste de stockage, et
- la mémorisation de l'emplacement de stockage du médicament en association avec l'identification de la référence du médicament.

Selon un mode de réalisation du brevet européen n° 1.176.106, les bandes sont munies (par exemple grâce à un dispositif de découpe par laser) de perforations destinées à recevoir respectivement les alvéoles des plaquettes de médicament. De par l'introduction des emplacements des doses unitaires sur la bande, il est possible en découpant la bande et la plaquette fixée dessus, de séparer individuellement les doses unitaires de médicament qui restent ainsi dans leur conditionnement primaire d'origine, sans aucun risque de découper ou briser une dose unitaire. Ce procédé est mise en oeuvre par un système comprenant :
- un poste de pré-conditionnement des plaquettes de médicament en bandes, ce poste comprenant des moyens (tels qu'une unité d'encollage) pour fixer les plaquettes de médicament sur un film en bandes,
- un poste de conditionnement des prises de médicaments, et
- un robot assurant la manutention des médicaments entre le poste de pré-conditionnement, le poste de stockage et le poste de conditionnement des prises, ce robot comportant des moyens pour découper le film et les plaquettes de médicaments collées dessus, de manière à en séparer au moins une dose unitaire de médicament.

Selon un système particulier, les plaquettes passent devant un lecteur optique permettant de lire un code à barre d'identification du médicament. Puis, les plaquettes sont appliquées sur la bande en passant entre deux rouleaux d'entraînement, la face où sont formées les alvéoles étant tournée vers la bande, et les alvéoles des plaquettes s'engageant dans les perforations de la bande. La bande avec les plaquettes collées dessus passe ensuite par une unité d'impression qui imprime sur chaque alvéole passant au travers de la bande, le code à barres lu par le lecteur optique, et de préférence la désignation en clair du médicament fournie en fonction du code à barres lu. La bande supportant les plaquettes de médicament collées est ensuite introduite dans une cartouche en attente à un emplacement de sortie du poste de conditionnement, en passant par une unité de découpe prévue pour découper la bande en un tronçon à la longueur de la cartouche.

Le robot pour la mise en oeuvre du procédé comprend une main comportant deux demi mâchoires supportant chacune une lame constituée de deux parties rectilignes formant un angle compris entre 45 et 135 degrés, les lames coopérerant l'une avec l'autre à la manière de ciseaux pour couper une plaquette fixée sur une bande.

WO 2010/095102 décrit un dispositif pour le traitement de marchandises unitaires (telles que des pilules ou comprimés) rangées dans un emballage (par exemple de type blister) dans lequel une unité de marchandise unitaire comprenant la marchandise unitaire et une partie de l'emballage entourant la marchandise unitaire est séparable du reste de l'emballage au moyen d'un dispositif de séparation, ledit dispositif de traitement comprenant un dispositif de marquage par lequel le marquage pour une identification univoque de l'unité de marchandise unitaire est ajouté à celle-ci. Selon un mode de réalisation, ledit dispositif de traitement comprend une unité d'enregistrement pour enregistrer de l'information relative à la marchandise unitaire (en particulier les caractéristiques de celle-ci et/ou une position de la marchandise unitaire dans l'emballage) à partir de l'emballage. L'unité d'enregistrement comprend par exemple un lecteur pour lire un code-barre et/ou une puce RFID et/ou un autre marquage présent sur l'emballage ou un suremballage. Selon un mode de réalisation, le dispositif de traitement comprend aussi un dispositif de fixation (par exemple point d'encollage, dispositif d'assemblage ou de soudure) permettant de connecter l'unité de marchandise à un élément d'entourage comprenant le marqueur. Selon un mode de réalisation, le dispositif de traitement comprend aussi un dispositif de coupage (tel que laser, poinçon, cisailles, ou jet d'air) pour produire des renfoncements de forme et/ou de taille adaptées à la marchandise unitaire. Selon un autre mode de réalisation, le dispositif de traitement comprend un dispositif (tel que laser, poinçon, cisailles ou jet d'air) pour séparer l'unité de marchandise unitaire du reste de l'emballage, auquel cas l'information relative à la marchandise unitaire et enregistrée dans l'unité d'enregistrement est dirigée vers le dispositif de séparation de façon qu'une séparation conforme à l'information enregistrée soit possible. La marchandise unitaire ainsi séparée est alors réceptionnée entre les éléments d'entourage.

Cependant, en pratique les systèmes automatisés et sécurisés de délivrance nominative de doses unitaires de médicaments traçables disponibles sur le marché nécessitent un investissement élevé (environ 1 million d'euros par système) et sont difficilement intégrables ou adaptables aux modes de fonctionnement de l'institution collective chargée de les recevoir et les faire fonctionner correctement. En outre, souvent ces systèmes sont essentiellement basés sur la découpe des plaquettes et l'application d'un suremballage (par exemple un sachet) qui augmente les temps de manipulation du personnel soignant. En outre, des systèmes automatisés de délivrance comportant l'emballage en sachets de médicaments déconditionnés ou livrés en vrac dans des bocaux, ne sont pas conformes à de nombreuses réglementations nationales, en particulier en France.

Un important problème des systèmes connus réside dans le fait que ces systèmes ne garantissent pas une relation univoque entre l'alvéole, séparée du blister et le médicament dans son conditionnement nouveau. Les alvéoles sont mélangées et le médicament, tel qu'indiqué dans le nouveau conditionnement risque de correspondre à un médicament sorti d'une autre boîte, avec par exemple une autre date de péremption ou à un tout autre médicament.

L'objet de la présente invention concerne un système automatisé entièrement sécurisé, utilisable dans une institution collective faisant appel à une pharmacie centrale, pour la délivrance nominative de conditionnements unitaires de médicaments aux patients, permettant une excellente traçabilité de ces conditionnements unitaires jusqu'à leur délivrance aux patients, permettant éventuellement sa mise en relation avec l'informatisation de la prescription desdits médicaments, et constitué d'éléments ayant une ergonomie acceptable pour le personnel de l'institution collective chargé de son utilisation.

### RÉSUMÉ DE L'INVENTION

Le but de la présente invention est de prévoir un système automatisé apte à la délivrance sécurisée d'une dose unitaire d'un médicament à partir d'une plaquette comprenant au moins une alvéole contenant le médicament. Le système comprend des moyens pour fixer une agrafe sur la plaquette, des moyens pour positionner et orienter cette agrafe par rapport à la plaquette et des moyens pour enregistrer des données concernant le positionnement et l'orientation des alvéoles sur la plaquette et/ou des données concernant le positionnement et l'orientation des sillons séparant les alvéoles sur la plaquette ainsi que des données concernant l'identification de la plaquette ainsi que des données concernant l'identité, position et orientation de l'agrafe attachée à la plaquette.

L'agrafe peut être orientée selon l'axe d'un des sillons séparant les alvéoles et/ou positionnée en deux dimensions suivant les caractéristiques géométriques de la plaquette d'alvéoles.

L'agrafe peut comprendre des moyens pour enregistrer des données concernant le positionnement et/ou l'orientation des alvéoles sur la plaquette et/ou des données concernant le positionnement et l'orientation des sillons séparant les alvéoles sur la plaquette et/ou des données concernant l'identification de la plaquette.

Les données concernant le positionnement et/ou l'orientation des alvéoles sur la plaquette et/ou des données concernant l'identification de la plaquette, peuvent servir à piloter la découpe et/ou le traitement des alvéoles qui y sont prélevées.

Le système automatisé peut comprendre en plus des moyens pour découper au moins une alvéole de ladite plaquette dans lequel la plaquette et les moyens de découpage sont positionnés l'un par rapport à l'autre suivant les données enregistrées dans l'agrafe.

L'appareil pour la fixation des agrafes et celui pour le découpage une à la fois de plaquettes tenues par une agrafe peuvent être dissociés.

Le système automatisé peut être muni de moyens pour libérer la plaquette de l'agrafe.

Le système automatisé peut comprendre en plus des moyens pour maintenir la position et l'orientation de l'alvéole pendant et après le découpage.

Les moyens pour maintenir la position après le découpage peuvent être constitués par au moins une zone en dépression agissant sur la superficie de l'alvéole découpée et, individuellement, ne dépassant pas cette superficie.

Le système automatisé peut comprendre en plus des moyens pour déplacer l'alvéole découpée en conservant l'information concernant la position et l'orientation de l'alvéole telle que découpée.

Les moyens pour déplacer l'alvéole peuvent être aptes à déplacer l'alvéole vers une position et orientation sur une carte, cette position et orientation étant déterminée par rapport à une fente, pratiquée dans la carte.

Le système automatisé peut comprendre en plus un poste d'encollage et les moyens de déplacement peuvent positionner l'alvéole devant le poste d'encollage pendant le trajet vers la position de fixation sur la carte.

Le poste d'encollage peut occuper une zone plus basse que le trajet directe vers la position de fixation sur la carte et les éléments mécaniques du système peuvent être substantiellement placés plus haut que cet endroit, à l'abri de projections de colle. Le système automatisé peut comprendre des moyens pour apporter des informations identifiant le médicament sur la carte après la découpage de l'alvéole contenant le médicament.

Le système automatisé peut comprendre des moyens pour empêcher un nouveau cycle de découpage et fixage de l'alvéole avant que l'alvéole précédente ne soit fixée sur la carte.

Le système automatisé peut comprendre des moyens pour stocker, en forme de bande, des cartes auxquelles doivent encore être apportées des informations identifiant un médicament.

L'axe des moyens pour stocker les cartes en forme de bande peut être disposée à l'écart de l'endroit ou se produit le découpage des alvéoles.

Le système automatisé peut comprendre des moyens pour ranger les doses unitaires une à une, dans l'ordre de leur production, dans un étui prévu à cet effet.

L'étui de rangement peut ne contenir qu'un type de médicament

L'étui de rangement peut être amovible et peut être destiné à servir pour la dispensation des médicaments contenus, auprès d'un service de soins.

Selon un autre but de la présente invention, une méthode est prévue pour la délivrance sécurisée d'une dose unitaire d'un médicament à partir d'une plaquette comprenant au moins une alvéole contenant le médicament telle que décrite ci-dessus. La méthode comprend les pas suivants: la fixation d'une agrafe sur la plaquette; le positionnement et l'orientation de cette agrafe; l' enregistrement des données concernant le positionnement et l'orientation des alvéoles sur la plaquette et/ou des données concernant le positionnement et l'orientation des sillons séparant les alvéoles sur la plaquette ainsi que des données concernant l'identification de la plaquette ainsi que des données concernant l'identité, position et orientation de l'agrafe attachée à la plaquette.

Au moins une partie des données enregistrées peut être enregistrée sur l'agrafe.

La méthode peut comprendre en outre l'alignement d'une rangée d'alvéole sur la plaquette par rapport à une des branches du couteau utilisé pour découper l'alvéole de la plaquette et le découpage de l'alvéole de la plaquette d'après les données enregistrées sur la disposition, par rapport à l'agrafe, des alvéoles sur la plaquette. La méthode peut comprendre en outre la fixation de l'alvéole découpée et/ou l'impression des données concernant l'identité sur une carte, cette fixation et/ou impression n'étant exécutée qu'après une confirmation qu'il n'y a pas de doute sur l'identité de l'alvéole à fixer.

La méthode peut comprendre une des opérations suivantes: l'impression des données identifiant le médicament sur la première carte en tête d'une bande de cartes provenant d'une réserve, le mouvement de la bande avec la carte imprimée en tête, vers le poste d'assemblage, la fixation de l'alvéole sur la carte ou le mouvement en sens inverse de la bande de carte.

La méthode peut comprendre au moins une des opérations suivantes: séparer la carte portant l'alvéole de la bande et rétracter la bande de cartes afin de présenter la carte en tête de bande en amont du poste d'impression.

### BREVE DESCRIPTION DES FIGURES :

Figures 1a et 1b : plusieurs vues d'une carte avec disposition de la fente et de l'alvéole, avant et après la fixation d'une alvéole ;
Figure 2 : différentes états d'un blister : plat et courbé ;
Figure 3 : différents arrangements des alvéoles et des sillons sur un blister ;
Figure 4 : découpage des alvéoles par un couteau ayant une forme en L ou par un couteau ayant une forme en T ; couteaux prévus d'une pointe ;
Figures 5a, 5b et 5c : plaquette avec une agrafe ;
Figures 6 : poste de chargement des plaquettes ;
Figure 7 : exemple de système de déplacement de l'alvéole.

### DESCRIPTION DETAILLEE DE L'INVENTION

Plus spécifiquement, selon un principe de base de l'invention, chaque plaquette d'alvéoles est d'abord munie d'un support de chargement, encore désigné "agrafe" qui peut comporter l'ensemble des informations signalétiques. Par ailleurs, on prépare des cartes aptes à recevoir au moins une partie de cet ensemble d'informations signalétiques, puis on prélève dans chaque plaquette les alvéoles contenant les doses unitaires de médicament et on fixe une carte signalétique à chaque alvéole ainsi prélevée sans déconditionner le médicament mais en transférant l'ensemble des informations signalétiques de l'agrafe ou d'une autre source vers chaque carte signalétique. La carte peut donc être utilisée comme véhicule de la dose unitaire de médicament et, en raison de sa conception ergonomique, permet notamment une extraction facile de la dose unitaire au moment de l'administration au patient par le personnel soignant.

Le système automatisé selon la présente invention permet de travailler en temps masqué, en respectant au maximum la flexibilité de l'organisation du personnel autour de la machine. Le système automatisé selon la présente invention, de par sa conception modulaire, permet que l'opération des différents modules puisse être séparée aussi bien géographiquement que dans le temps.

Selon un mode de réalisation particulier de la présente invention, la fixation d'une agrafe dotée d'une puce d'identification par radiofréquence (RFID) permet de donner une identité unique à chaque plaquette de médicaments à intégrer dans le système. Cette agrafe peut être fixée sur la plaquette à un poste de travail alimenté par un opérateur (par ex. un préparateur de la pharmacie) qui contrôle la qualité et l'intégrité des médicaments avant leur transformation. Ce poste de travail de fixation de l'agrafe, est de préférence relié à une ou plusieurs banques de données concernant le médicament, assiste l'opérateur dans son contrôle et valide l'entrée en allouant, à la puce RFID dans l'agrafe, le code unique qui servira au repérages successifs du médicament tout au long de son entreposage ou sa transformation. La pose de l'agrafe se fait à un rythme et dans une période qui n'est pas nécessairement directement liée avec la production des cartes signalétiques. De ce fait l'agrafe permet autant la manipulation automatique par un robot emmagasineur que l'entreposage par voie manuelle ou encore son passage direct vers l'appareil produisant les doses unitaires identifiées.

Ce module au coeur du système, producteur de doses unitaires sans déconditionner le médicament, est entièrement automatique. Il prélève dans les plaquettes les alvéoles contenant les médicaments, prépare les cartes signalétiques et les y fixe sans déconditionnement, puis les présente rangées dans un dispositif de rangement tel qu'un étui.

L'approvisionnement de ce module central peut se faire manuellement ou automatiquement. Dans les deux cas les plaquettes de médicaments ont étés préalablement munies d'une agrafe, pouvant contenir l'ensemble des informations nécessaires et suffisantes pour reconnaître et tracer le médicament.

L'appareil lit cet ensemble d'informations et les transfère individuellement à chacune des cartes signalétiques sur laquelle est fixée l'alvéole avec la dose unitaire du médicament correspondante.

La production de l'appareil sort sous forme de dispositifs de rangement (par exemple étuis) remplis de cartes signalétiques portant sur chacune d'elle une alvéole et la nomenclature du médicament. Ces étuis peuvent avoir des formes différentes adaptées à leur destinataire. Généralement, la production de l'appareil est entreposée avant d'être dispensée pour délivrance aux patients. Dans un système entièrement automatisé, la mise en rayon de stockage des dispositifs de rangement (étuis) remplis se fait par le moyen d'un robot emmagasineur.

Le système automatisé selon la présente invention, de par sa conception modulaire, permet que l'opération des différents modules puisse être séparée aussi bien géographiquement que dans le temps. Définitions : dans la présente description et dans les revendications y attachées, les définitions suivantes sont valables : Dose unitaire : une alvéole individuelle avec un médicament, attachée à une carte et comportant des informations signalétiques du médicament ; normalement la dose unitaire comprend toutes les informations nécessaire pour la traçabilité et est destinée à être utilisée en une seule fois. Alvéole : un élément, généralement fermé, comportant un petit logement dans lequel est logé un médicament, le petit logement étant fermé au dessus par une cloche et en dessous par une opercule (l'envers de l'alvéole) Système de transformation : un système qui « transforme » un blister, comportant une ou plusieurs alvéoles, dans une série de doses unitaires, tout en maintenant la traçabilité de la dose unitaire. Axe principal d'une alvéole : en général, l'axe de symétrie le plus long de l'alvéole ou du logement se trouvant sur l'alvéole , dans le cas où il y a plusieurs de ces axes (p.ex. le cas d'un logement rond), un quelconque de ces axes. Délivrance/préparation sécurisée : la délivrance/préparation d'une dose unitaire de façon à ce qu'il y a une relation sans équivoque entre chaque alvéole, faisant partie d'une dose unitaire et une alvéole, qui était présente sur un blister. Il faut aussi noter que le verbe « comprendre », utilisé dans la description et dans les revendications, ne peut pas être interprété d'une façon restrictive en relation avec les éléments ou pas, énumérés après le verbe. Le verbe est à interpréter comme une indication que ces éléments ou pas sont présents, ce qui n'exclut nullement la présence d'autres éléments ou pas.

L'ensemble du système sera décrit expliquant les différentes étapes et en détaillant les différents modules. A noter que toutes les étapes ne sont pas toujours nécessaires ; l'invention couvre aussi des systèmes ou des méthodes dans lesquels certains étapes ne sont pas présentes.

### Chargement des plaquettes.

Cette opération, éventuellement manuelle, peut être faite p.ex. à partir de cartons contenant une variété de produits emballés dans des boites (de tailles et formats différents) appelées emballages secondaires. Sur les boîtes peuvent être imprimées des informations en toutes lettres et/ou en code-barre et/ou -matriciel, des informations concernant le contenu de chaque boîte tel que le nom, le principe actif, la date de fabrication, le numéro de lot de fabrication, la date de péremption, le nom du fabricant, le contenu de la boite en nombre de doses individuelles et le dosage de chacune de ces doses.

Ces informations prises ensemble identifient complètement le médicament et sa provenance. Certaines informations sont légalement requis pour former une identification suffisante et sans équivoque du médicament pour sa traçabilité lors de sa dispensation.

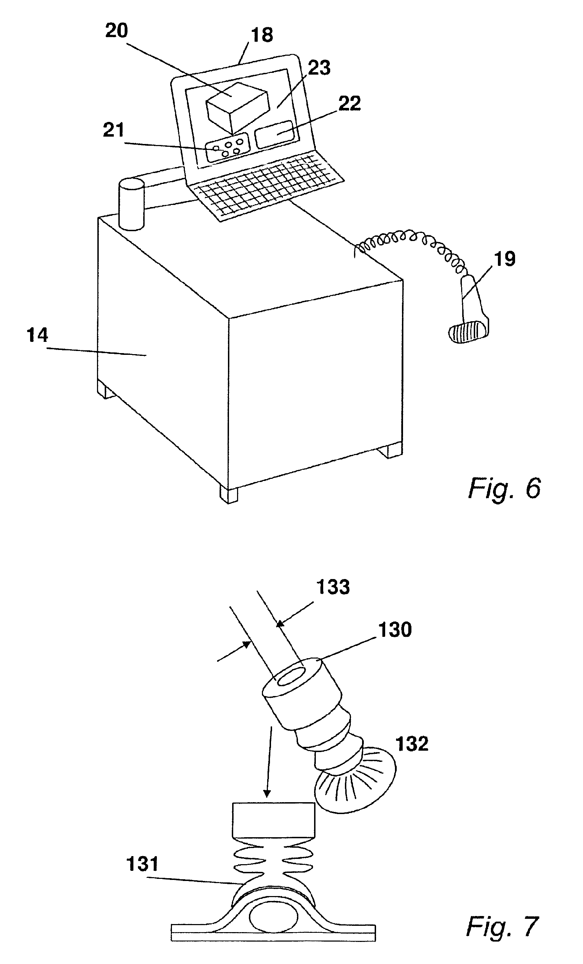

Contrôle de la correspondance de la boite de plaquettes avec les critères de sélection. (Fig. 6) Les informations, qui suffisent pour identifier le médicament et son applicabilité sans équivoque, devront accompagner chacune des doses, jusqu'à leur moment de consommation pour assurer une traçabilité complète du médicament lors de son stockage, sa dispensation et de son administration.

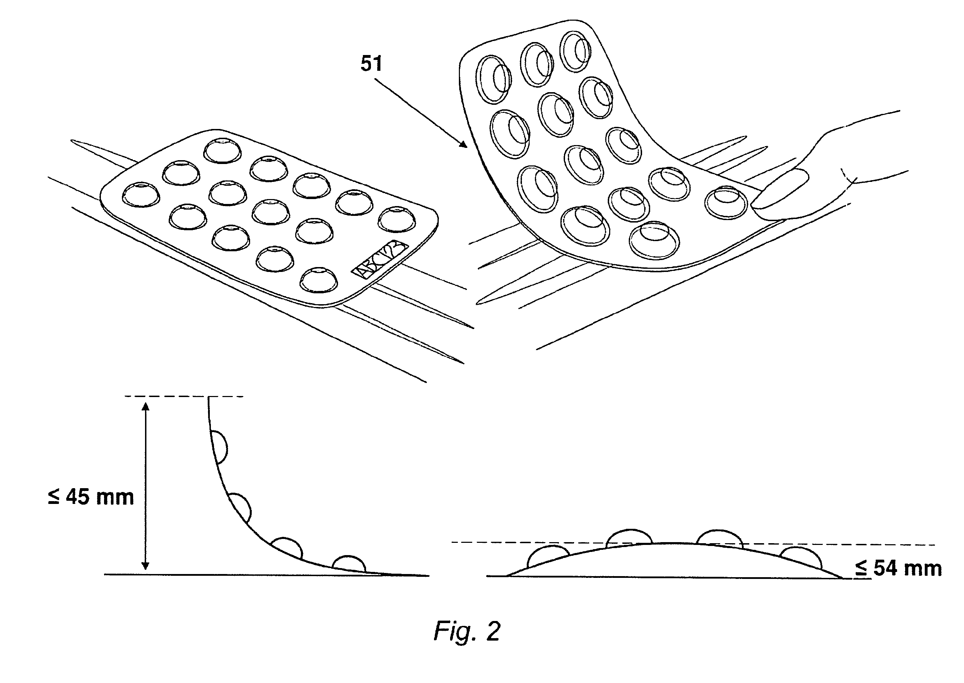

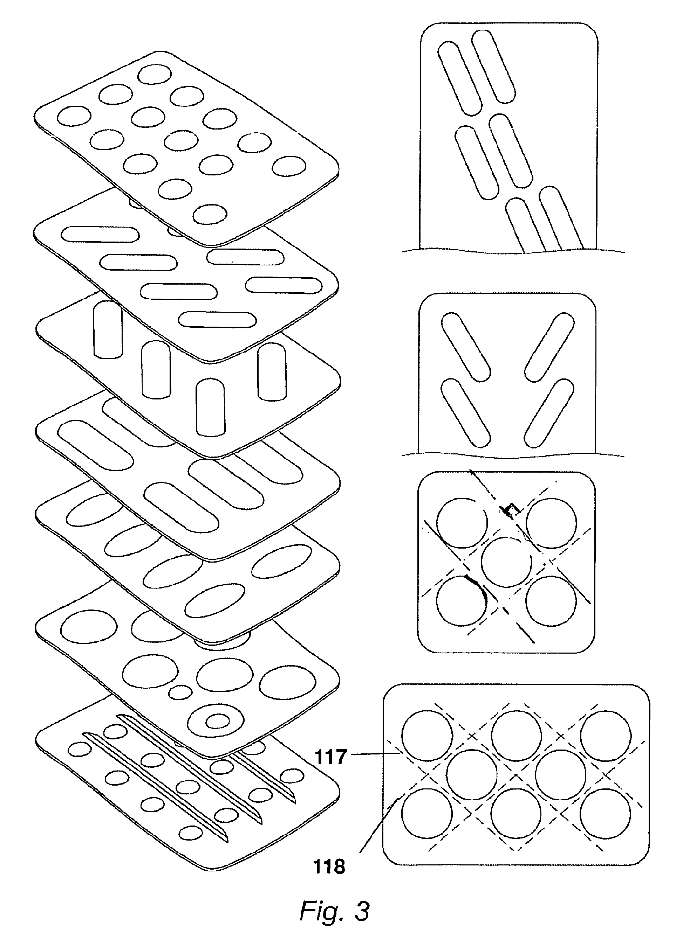

Chaque boîte peut contenir des plaquettes (Fig. 2 et 3) d'alvéoles (1) (au moins une plaquette et au moins une alvéole par plaquette). Le conditionnement du médicament est intact tant que l'atmosphère extérieure reste exclu de l'intérieur de l'alvéole (15) contenant le médicament. L'emballage primaire est l'enveloppe protectrice du médicament formée par les parois (16)(17) intacts de l'alvéole.

Pour capter les informations sur le contenu des emballages primaires, l'appareil de contrôle peut être prévu d'un pupitre (18) d'entrée de données manuelle ou les données peuvent être transcrites de la boite par un capteur (19) lecteur de code-barres.

C'est la responsabilité de la personne chargeant l'appareil (14) de s'assurer que les informations stipulées sont bien présentes sur l'emballage secondaire et qu'elles sont cohérentes avec le contenu de l'emballage secondaire .

Paramétrage de la réception du nombre de plaquettes de la boite (Fig. 6)

Afin de faciliter la tâche de l'operateur,_une image (20) de la boite (par ex une simulation tournante en 3D) et des deux faces (21,22) du type de blister (Fig. 3) qu'elle contient peut être projetée sur un écran (23) à proximité.

Le repérage du type de médicament peut être fait par rapport à une banque de données contenant les caractéristiques marquantes et utiles définissant les paramètres, nécessaires à l' opération du système.

Sur toutes les plaquettes sont disposées d'une façon ni standardisée ni facilement lisible, toutes informations légalement requises pour le repérage du médicament contenu dans les alvéoles de la plaquette. Généralement, certaines de ces informations ne se trouvent pas dans une zone ou sont concentrées les alvéoles, mais sont rejetées sur un des bords de la plaquette, généralement poinçonnées et lisibles uniquement du coté lisse. Toutes les alvéoles séparées d'une telle plaquette ne peuvent pas toutes être séparées avec le même bord poinçonné y encore attaché; il y aura donc des alvéoles séparées , démunies d'identification sans équivoque ou avec des renseignements incomplets sur leur contenu. Dans un tel cas, l'alvéole et la pilule logée dedans et les textes imprimés dessus, ne sont plus considérés fiables pour son introduction dans le circuit médicamenteux . De telles plaquettes doivent donc rester entières le plus longtemps possible.

Dans certains cas, l'ensemble de ces informations identifiantes sont répétées, à nouveau d'une façon ni standardisée ni facilement lisible, sur la zone de la plaquette où se trouvent les alvéoles mêmes . La zone est délimitée par sa contigüité avec les zones avoisinantes contenant d'autre alvéoles. Souvent, cette délimitation est prévue d'une perforation , permettant facilement la séparation d'une alvéole de la plaquette. Dans un tel cas, une alvéole porte toutes les informations suffisantes pour son identification médicamenteuse et même séparée de la plaquette, pourrait entrer dans le circuit médicamenteux sans encombre. Toutefois, la forme et la présentation de ces alvéoles de dose unitaires, n'étant pas standardisée, le personnel qui doit en assurer la dispensation et administration et les contrôles intermédiaires est aussi gêné dans son activité.

La lecture des informations identifiants sur chacune des plaquettes ou au dos de chacune des alvéoles ne se fait pas en détail lors de la mise en magasin des médicaments: le personnel responsable se fiant aux informations sur l'emballage secondaire et sur la vigilance du personnel en aval pour contrôler au possible l'emballage primaire au moment de la dispensation et l'administration au patient. Il en résulte des erreurs, qui pourront être évitées si la présentation du médicament lors de sa dispensation et de son administration au patient est complète et facilement lisible ou contrôlable. Les éléments du système proposé reposent sur un processus pour présenter sous forme ergonomique et standardisée tout médicament pris en compte en dose unitaire identifiée individuellement lors de sa préparation sans le déconditionnement de son emballage primaire.

Seuls, les médicaments répertoriés dans la banque de données du système peuvent être traités automatiquement par le système. La banque de données est alimentée par des informations, préparées au préalable et statistiquement assurées puisque relevées sur une quantité de plaquettes du même type de médicament, présentées dans la même forme d'emballage secondaire. Chaque modification de la présentation peut générer une nouvelle référence dans la banque de données et donc, aussi, de nouvelles images de contrôle pour l'utilisateur.

L'alternative du repérage de la position des alvéoles sur les plaquettes une à la fois ou lors de leur entrée dans l'appareil, s'avère non seulement fastidieuse, très longue à mettre en oeuvre, peu productive, mais aussi risquée si elle n'est pas faite de manière consistante et par du personnel formé et equipé. L'opérateur ayant reconnu le produit peut confirmer qu'il veut le charger; un « décompteur » du nombre de blisters ou plaquettes attendues dans la boite d'emballage secondaire peut être prévu..

Contrôle de la correspondance de chaque plaquette introduite avec les critères de sélection.

Une des particularités des blisters, ou plaquettes d'alvéoles, est la variété entre les médicaments.

Ces caractéristiques combinées avec le coloris des médicaments et de l'une ou l'autre des faces de la plaquette, servent à aider l'operateur à différencier rapidement et visuellement les médicaments l'un de l'autre.

A titre d'exemple, parmi 500 plaquettes observées en officine de pharmacie, on a trouvé 444 formes différentes d'emplacement des alvéoles (écartement et disposition sur la plaquette), de géométrie des alvéoles (longueur, hauteur, largeur) et d'orientation des alvéoles (par rapport aux axes principaux de la plaquette).

Les dimensions des plaquettes variaient ainsi que la hauteur de cloche d'alvéoles. Les cloches d'alvéoles ont des formes différentes : circulaires, ovales ou ellipsoïdales. L'axe majeur des cloches non circulaires est orienté à un angle qui varie entre 0° et +90° et -90° par rapport à l'axe principal du bord extérieur de la plaquette et la disposition des cloches n'est pas nécessairement à un même écart, régulier sur toute la plaquette. Il existe donc une grande variation dans la disposition et la forme des alvéoles (voir les exemples Fig. 3).

Entre plaquettes d'un seul médicament, il y a régularité, qui sera mise à profit pour le traitement ultérieur dont suit la description.

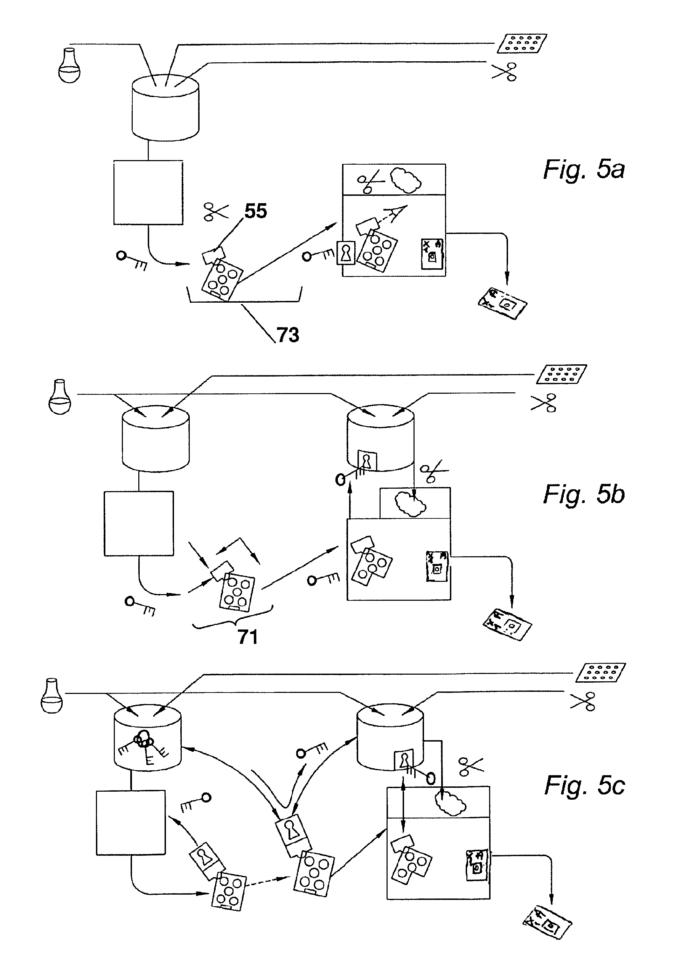

Introduction des plaquettes sur un support de chargement (l'agrafe) (Fig. 2, Fig. 5)

La géométrie très variable rend le traitement et/ou la transformation par un système automatisé compliqué.

Les plaquettes, sorties de leur emballage secondaire et posées coté lisse sur une table, ne sont pas nécessairement complètement plates mais peuvent avoir un profil en courbe (51). Pour éliminer les risques d'erreurs, la plaquette peut être introduite jusqu'à ce qu'elle arrive en butée au fond d'un goulot qui la rend plane. Après, un élément mécanique et standardisé, appelé « support de chargement » ou encore « agrafe » (55) peut être fixé à la plaquette. Les flancs , surfaces ou formes extérieures des agrafes peuvent être pourvus des points d'appuis mécaniques, nommés « registres » permettant un positionnement déterminé et précis par rapport à une autre pièce ou support ayant la contre-forme adaptée.

Positionnement et orientation du support de chargement en fonction des paramètres spécifiques de la plaquette. (Fig.3) Afin que l'appareil puisse traiter et transformer les plaquettes et en séparer les alvéoles, la position et l'orientation exacte des alvéoles doivent être connues et/ou la position et l'orientation des sillons séparant les alvéoles .

Dans ce but, il est avantageux d'aligner les registres du support de chargement (flancs de l'agrafe) sur l'orientation des alvéoles (plutôt que sur l'orientation des axes principaux de la plaquette) ou sur l'orientation des sillons séparant les alvéoles. Pour ce faire, le poste de chargement peut être pourvu d'un axe-motorisé, qui oriente le support de chargement. Pour un positionnement correcte d'un objet de forme essentiellement rectangulaire, tel qu'une plaquette, le contact avec trois butées suffit. L'invention prévoit un contrôle automatique au niveau de chacune des butées lors du chargement et le support de chargement n'est fixé à la plaquette que lorsque celle-ci est en butée avec les trois repères. Le support de chargement peut être fixé à la plaquette d'une façon indissociable. Vu que l'orientation et la position relative de la plaquette et du support de chargement sont fixées l'un par rapport à l'autre, les flancs de l'agrafe peuvent faire office de registre de positionnement pour le repérage individuelle de chacune des alvéoles sur la plaquette. Les tolérances de positionnement des bords de la plaquettes par rapport aux alvéoles sont suffisamment serrées pour que la séparation des alvéoles par découpage individuel de la majorité de plaquettes puisse être faite sans difficultés. Pour les plaquettes présentant des sillons de séparation entre les alvéoles de largeur réduite et dans les cas ou ces tolérances pourraient poser problème, une mesure de la position des rangées d'alvéoles sur la plaquette peut être nécessaire. Dans ce cas, le découpage des alvéoles devrait se faire en fonction de la mesure prise sur la plaquette. Généralement, l'agrafe peut être pourvue d'un enregistrement des données concernant la position des alvéoles. Cet enregistrement peut être un code bar, un code matriciel à 2 Dimensions, une puce électronique, RFID ou tout autre moyen de lecture/écriture. Cet enregistrement pourrait contenir toute information concernant la géometrie, la manipulation et le découpage de la plaquette tenue par l'agrafe. Cet enregistrement rend possible le traitement des plaquettes sans connexion à une banque de données répertoriant les caractéristiques de tous les médicaments en considération.

Le positionnement de l'agrafe sur la plaquette est faite en tenant compte de la position des alvéoles, présentes sur la plaquette. Ainsi sont évitées des difficultés lors du découpage ultérieur de ces alvéoles. En effet, la présence de l'agrafe pourrait rendre le découpage difficile, voir même impossible pour certaines alvéoles. Le positionnement de l'agrafe est donc fait de façon à réduire au maximum les difficultés possibles lors du découpage.

Marquage individuel de l'ensemble de la plaquette et du support de chargement afin de l'identifier

Chaque plaquette est dotée d'une identité, puisqu'elle sort d'un emballage secondaire bien particulier, tandis que le support de chargement contient également des données spécifiques.

Ces informations et données forment un identifiant unique que le système peut accorder à l'ensemble support de chargement et plaquette (71); cet identifiant peut être mis en mémoire pour pouvoir repérer ultérieurement la plaquette et également chacune des alvéoles qu'elle comporte.

Le moyen choisi pour conserver ces informations à bord de cet ensemble peut être un RFID contenu dans le support de chargement. Le RFID a son propre code identifiant unique. La lecture de celui-ci permet de retrouver dans la mémoire de l'appareil le fichier rapportant à la plaquette concernée. Pour éviter qu'il puisse-t-y avoir de la confusion entre deux plaquettes ayant un même code RFID (par exemple lorsque le même code est réutilisé dans une succession de plaquettes), un RFID programmable permet l'ajout de suppléments, identifiant le temps des étapes.

Transfert de l'ensemble hors zone de chargement (pour stockage intermédiare ou traitement directe d'emballage)

A partir du moment où le contenu de la boite est réparti en un nombre de plaquettes, chaque plaquette a une identité unique, qui se réfère à la boite d'origine. Cet élément traçable peut être entreposé dans un magasin (73) pour une dispensation ultérieure ou une transformation différée. Le cas échéant, moyennant une lecture de la puce RFID dans le support de chargement, les alvéoles, de la plaquette ainsi identifiée, peuvent être elles-mêmes aisément identifiées lors d'une séparation manuelle telle qui se fait couramment dans les services hospitaliers. Même une plaquette, dont on aurait enlevé manuellement une quantité d'alvéoles, peut être réintégrée au suivi du système de manière sécurisée, pour autant que le nombre et positions d'alvéoles restantes sur la plaquette soit mémorisé dans la puce RFID, ce qui peut se faire avec une reprogrammation locale de la puce. Les nouvelles informations peuvent être connues de la mémoire centrale par lecture de la puce RFID reprogrammée à tout moment voulu.

Alternativement, l'ensemble peut être directement introduit au procédé de remballage décrit plus loin.

Emballage des alveoles- Lancement d' une campagne d'emballage d'alvéole(s) ou de transformation. L'appareil d'emballage des alvéoles peut être commandé sur demande ou agir de façon automatique. Pour fonctionner, les sécurités internes de l'appareil (tension, température de colle, cartes en réserve, encre en réserve, air sous pression, portes de sécurité fermées, etc.) doivent permettre le démarrage de l'appareil d'emballage ou de « transformation ». Avant le lancement d'une campagne, la combinaison de présence d'une plaquette, d'une commande de transformation, d'un étui pour stocker les produits finis doit être satisfaite. La priorité sera de manière générale donnée à la terminaison de la tâche en cours, mais des interruptions doivent aussi être possibles, sans perte de sécurité. Le manque d'un de ces éléments doit provoquer l'interruption et le verrouillage de l'activité. La remise en route et continuation de la tache après une interruption se fera automatiquement lorsque les conditions de marche sont à nouveau réunies.

La commande de transformation peut définir un nombre entier de plaquettes à transformer ou un nombre d'alvéoles, qui peut dépasser le nombre contenu sur une seule plaquette..

Sélectionnement d'une plaquette à transformer. A partir de la commande de transformation (informatisée ou introduite au clavier de l'appareil), la plaquette ou un ensemble agrafe-identifiée avec sa plaquette portant les médicaments correspondants peut être extraite du magasin.

Le magasin peut avoir des formes allant d'un système séquentiel tel qu'un carrousel de chargement ou d'une file d'attente sur un rail au système parallèle d'un magasin de niches , d'accès libre. Dans tous les cas, le choix pour traitement commençant par la plaquette la plus ancienne est a privilégier, car ce faisant, le risque de stocks périmés est le mieux maitrisé. Lorsque le magasin est séquentiel, il est important que la sortie du magasin se fasse au bout opposé à son entrée .

La plaquette est repérée par lecture de la mémoire des mises en magasin ou bien par recherche de l'identifiant, programmé dans le support de chargement, parmi les plaquettes gardées en stock.

L'ordre de chargement d'un magasin séquentiel peut en déterminer l'ordre de sortie et de transformation. L'opérateur peut déterminer quel sera l'ordre de transformation en posant les plaquettes et leur agrafe à la bonne séquence suivant la direction de rotation d'un magasin en carrousel. Cela implique qu'un appareil même dépourvu de programmation peut exécuter des transformations pour autant qu'il puisse lire le contenu des RFID dans les agrafes.

### Prélèvement des identifiants spécifiques à la plaquette

Lorsque la plaquette est sélectionnée, la correspondance correcte des informations la concernant et les critères de sélection, définis par la commande, peut être vérifiée et les identifiants peuvent être tenus en mémoire temporaire de l'appareil pendant le séjour de traitement de la plaquette. L'aspect sécuritaire est assuré avec l'agrafe à puce RFID reprogrammable par le fait que la machine faisant la transformation ne doive pas se fier uniquement à des fichiers informatiques qu'elle détient seule pour relier la transformation à la plaquette, mais pourra lire le programme porté dans l'RFID et en double sécurisation, en contrôler la cohérence avec ses propres fichiers.

Extraction des paramètres de transformation de la plaquette avec ses alvéoles pour procéder au découpage, au marquage et à l'emballage

Afin de transformer la plaquette, l'appareil doit charger les paramètres de transformation mis en mémoire, qui correspondent au type de la plaquette. La mémoire peut être une banque de données ou une mémoire (temporaire) alimentée par l'operateur afin de pouvoir procéder à un découpage. Le RFID peut tenir toute information utile concernant la plaquette, y compris les instructions de transformation (découpage) de la plaquette.

La transformation peut être exécutée à partir des informations contenues dans le RFID, sans devoir faire référence aux fichiers de la machine ayant programmée l'agrafe.

Il est aussi possible avec ce système, qu'une machine de transformation, géographiquement séparée puisse à n'importe quel moment ultérieur y ajouter les informations concernant les opérations qu'elle doit faire ou l'opération faite sur la plaquette.

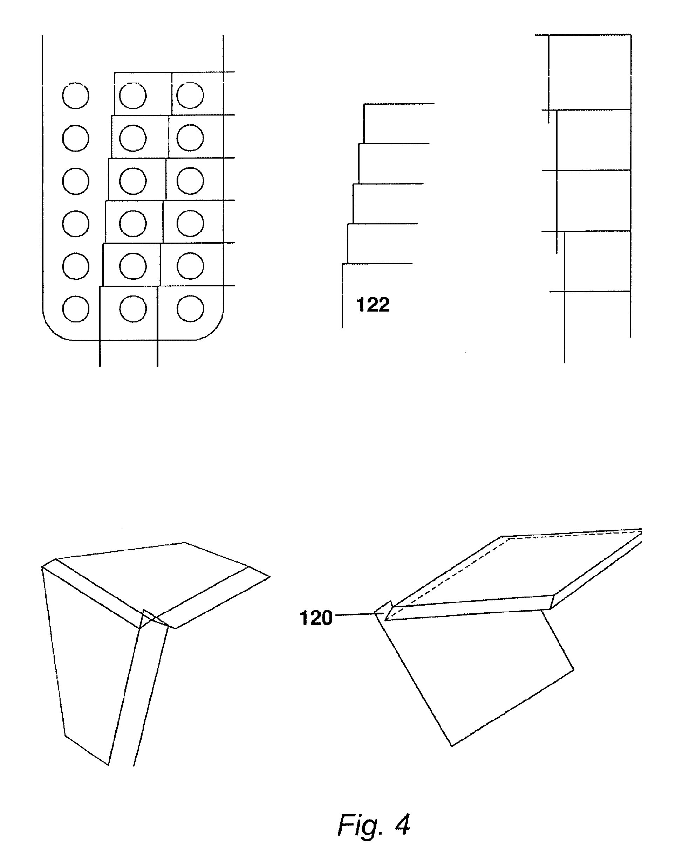

Découpage optimal de la plaquette fonction de la forme des ciseaux utilisés. (Fig. 4). Lors d'un découpage manuelle, les ciseaux sont généralement droits. Il y a plusieurs façons de découper, toutes aboutissent à contourner l'alvéole à séparer. Par exemple, il est parfois possible de décrire une courbe partant d'un bord de la plaquette, autour de l'alvéole et sortant de la plaquette sur un autre bord. Cette façon demande un espacement assez large entre les alvéoles. Pour séparer manuellement une alvéole de la plaquette, deux entailles rectilignes suffiront, pour autant qu'elles se rencontrent en un point ou qu'elles se croisent tout en contournant l'alvéole. Ces entailles sont faites successivement et partant de deux bords extérieurs de la plaquette, l'un pratiquement à angle droit par rapport à l'autre. Une machine peut réaliser un tel découpage, s'il est équipé de couteaux rectilignes, mais obligatoirement, les découpages sont faits l'un après l'autre. Si, par contre, l'appareil est équipé d'un seul couteau en forme de L, les deux entailles pourront être faites en un mouvement pour autant que le coin du L est positionné à l'endroit où les entailles du découpage manuel se rencontrent. Une telle configuration a l'avantage d' augmenter la productivité du découpage (plus que doubler). Il est clair que prélever une série d'alvéoles d'une plaquette demande une série de découpages successifs._La position du deuxième découpage par rapport à la première devra logiquement aussi partir d'un bord (d'origine ou nouvellement créé) de la plaquette et pourrait théoriquement être faite en plaçant le coin du L à une distance d'une alvéole dans la direction de la prolongation d'un des bras du L. Toutefois, même une erreur minimale dans le positionnement aurait comme conséquence que le nouveau découpage ne communiquera pas avec le découpage précédent; la deuxième alvéole ne sera pas séparée de la plaquette et y restera attachée par une fine lichette auprès du coin de la précédente échancrure. Il est essentiel de remédier à ce problème. L'option de déplacer légèrement le deuxième découpage vers le bord extérieur de la plaquette, éviterait la formation des lichettes, mais après une série de découpages dans une rangée, le cumul de ces petits déplacements rapprocherait inévitablement le découpage vers le bord des alvéoles (voir Fig. 4, 122). L'espace entre les rangées d'alvéoles est souvent réduit et, compte tenu des tolérances de positionnement, ne permet que tout juste le passage de l'entaille en son milieu sans déborder dans la cloche d'une alvéole. La série de découpages faits comme décrit ci-avant forme un « escalier » qui part légèrement en biais par rapport à l'axe des entailles des L, qui doivent communiquer avec l'échancrure précédente. *Moyennent une rotation du L du couteau, afin de réaligner le biais avec la rangée d'alvéoles, l'effet cumulatif peut être compensé.* Toutefois cela demande que l'espace libre entre les rangées d'alvéoles soit suffisamment large pour permettre une coupe en diagonale, mais, comme décrit plus haut, souvent cet espacement est insuffisant.

Le fait de former le L avec un angle légèrement inférieur à 90° pourraient assurer à ce que l'empiétement des découpages successifs est garanti et donnerait le même effet que la rotation précitée sans toutefois affecter l'orientation de l'autre jambe du L.

Manuellement, l'empiétement d'un deuxième découpage sur le précédent est obtenu naturellement puisque ce découpage partira depuis le coin intérieur de l'échancrure précédente. Le découpage ne se fait pas entièrement rectiligne mais « attaquera » un peu vers l'intérieur pour ensuite se redresser parallèle à l'ancien découpage. Un couteau en L, dont les extrémités des bras sont courbées vers l'intérieur tout comme un couteau en L dont les bras sont à un angle inférieur à 90°, doit obligatoirement être dimensionné en fonction des dimensions des alvéoles à découper. Il faut donc avoir des couteaux d'angles différents et de longueur de jambes différente pour réaliser tous les découpages.

Une autre solution utilise l'espace libre en forme d'étoile au croisement des sillons séparant les alvéoles. Puisque les alvéoles sont faites par une bulle déformée dans le plastique ou l'aluminium d'emballage des médicaments, les alvéoles ont une forme généralement arrondie sur les coins, ce qui rend l'espace entre les alvéoles à cet endroit plus large. A cet endroit sans perte de généralité, en travers de la rangée d'alvéoles, un débordement de l'entaille du découpage précédent par rapport à sa longueur minimum théorique est admissible sur au moins la largeur du sillon minimum ou de la tolérance de positionnement du couteau par rapport aux plaquettes. Ceci permet à un deuxième découpage dans l'axe de la rangée d'alvéoles, fait avec un couteau en T ouvert à 90°, d' empiéter et de communiquer avec le découpage précédent. Un couteau ayant une longueur suffisante pour découper les alvéoles les plus grandes, ne gênera pas lors de son application au découpage des alvéoles plus petites.

Avantageusement, on utilise un couteau en forme de T inversée ; la forme du couteau ressemble à un L dont la barre horizontale dépasse vers la gauche (ou la droite, selon le cas). Dans le cas d'un couteau en forme de T, une des branches horizontales peut posséder une longueur inférieure à l'autre branche. Le dépassement de la branche vers la gauche ou la droite peut être petit et il suffit qu'il soit long assez pour compenser le manque de précision de positionnement attendu : environs 0.5mm de dépassement suffiront alors que les jambes principales du L ont une longueur dépassant 35mm afin de contourner même les alvéoles les plus grandes.

Une combinaison des principes énoncés est parfaitement possible.

Comme les sillons séparant les alvéoles peuvent être étroits, ils peuvent aussi être irrégulièrement larges. Certains sillons peuvent être tellement larges que la zone de la plaquette associée à chaque alvéole est plus grande que l'espace prévue pour recevoir les alvéoles sur le support d'emballage (la carte), ou tels que les parties en dehors de la partie sous la cloche d'alvéole dépassent de plus de 5mm. Dans ce cas les plots de colle qui sont toujours à proximité de la cloche, ne soutiendront pas les parties de plaquettes dépassants (voir plus loin). Ces parties non soutenues peuvent être gênantes pendant des opérations ultérieures et pour la manipulation par l'infirmière lors de l'administration du médicament. Dans le cas de découpage de plaquettes selon des sillons en biais, il est inévitable que des alvéoles provenant de la proximité des bords de la plaquette aient des formes quasi triangulaires avec au moins un coin pointu à angle aigu de moins de 90°. Ces angles sont dangereux pour les infirmières et autres personnes manipulant de telles alvéoles découpées et peuvent provoquer des blessures.

Ces parties peuvent être enlevées par une action de découpage supplémentaire, laissant une alvéole de forme et de taille plus semblable à ses consoeurs et plus facile à manipuler. Les parties découpées sont des déchets qu'il n'y a pas lieu d'intégrer dans le reste du parcours de transformation. Lorsqu'elles sont découpées, une suppression de l'aspiration de l'enclume et/ou une inversion de l'aspiration, remplacée par un souffle, éjecte ces déchets. L'appareil sera avantageusement équipé de guides ou parois protégeant ses éléments mécaniques, afin que les déchets soient évacués sans s'y loger. La prévision d'un réceptacle amovible rendra plus aisée l'entretien et le maintien de la propreté de la machine.

On peut prévoir des découpages supplémentaires entre les découpages d'alvéoles pour donner une forme plus régulière et maintenue plus proche de la cloche de l'alvéole.

On peut aussi prévoir des guides et bacs pour l'évacuation des découpages, hors circuit de transformation

### Aplatissement des plaquettes

Pour aplanir les plaquettes courbes, il suffit de les glisser dans une fente dont les parois sont espacées de la hauteur des cloches d'alvéoles. A l'approche de cette fente, un goulot avaloir avec une ouverture assez grande pour recevoir les plaquettes courbées est à prévoir.

Selon l'invention, les plaquettes peuvent être aplaties en les glissant dans une fente dont les parois sont espacées de la hauteur des cloches d'alvéoles. A l'approche de cette fente, un goulot avaloir avec une ouverture assez grande pour recevoir les plaquettes courbées est à prévoir. En pratique il s'avère qu'un goulot dépassant les 50mm d'ouverture et muni d'un avaloir en entonnoir à pente ne dépassant pas 35° suffit à la tâche, même lorsque les plaquettes approchent avec la partie soulevée l'avant. Ce type d'aplanisseur convient à un processus automatisé, car le résultat est obtenu dans tous les cas pratiques de présentation des plaquettes.

Puisque les cloches d'alvéoles varient en hauteur de 1 à 11 mm, un espacement fixe des parois de la fente de 11 mm laissera plus de liberté à une plaquette avec des cloches d'alvéoles basses que lorsque celles-ci ont une hauteur de 11 mm. Par exemple, une cloche de 3mm de hauteur a encore 8mm libre et, compte tenu de la courbature naturelle des plaquettes, les alvéoles les plus éloignées de l'agrafe seront soulevées hors du plan théorique. Etant donné que ce sont les alvéoles les plus éloignées de l'agrafe qui sont les premières à découper, il sera avantageux, lors du découpage même, de forcer celles-ci contre une surface entièrement plate. Avec ce type d'aplatisseur, l'ouverture du couteau peut être minimisée pour toutes les plaquettes, sans devoir tenir compte de leur courbure naturelle initiale. Cette ouverture suffit d'être légèrement supérieure à la hauteur proposée du tunnel, soit >11 mm dans le cas décrit, au lieu de >45mm si les plaquettes les plus courbées devaient s'introduire dans le couteau.

Orientation de la plaquette contenant l'alvéole par rapport aux axes de référence du système (Fig. 3). Il existe une très grande variété de dispositions d'alvéoles sur les plaquettes et une infinité de dispositions possibles.

Bon nombre des plaquettes demandent des entailles orientées en biais (117,118) pour le découpage des alvéoles. Les angles ont une valeur entre -75°, 0° et +75° avec une concentration entre 30° et 60° et -30° et -60° par rapport au bords de la plaquette.

Que la séparation des alvéoles se fasse en un mouvement unique d' un couteau en L ou en T ou par plusieurs mouvements successifs, les entailles doivent être orientées substantiellement en alignement avec les rangées d'alvéoles (ou en alignement avec les sillons entre ces rangées) et donc en cas général pas nécessairement avec les bords des plaquettes. Cet alignement relatif se fera plus aisément en orientant la plaquette d'alvéoles par rapport aux axes du couteau de la machine que inversement.

Les flancs de l'agrafe montée sur la plaquette donnent un repère mécanique précis permettant d'orienter celle-ci, malgré son manque de rigidité et sa tendance à se courber hors de son plan.

Puisque l'agrafe a été fixée à la plaquette dans un appareil se repérant sur les bords extérieurs aplatis de la plaquette, la disposition correcte des alvéoles sera retrouvée si les flancs de repère de l'agrafe servent à la tenir à nouveau pendant le découpage et que en plus la plaquette est à nouveau aplatie.

Découpage et tenue d'une alvéole, afin qu'elle soit désolidarisé de la plaquette dont elle faisait partie, tout en conservant son orientation et sa position. (Fig. 4). Plusieurs méthodes de découpage ont été proposées; dans la plupart de ces méthodes la position et l'orientation de l'alvéole après découpage ne sont pas maintenues, mais l'alvéole tombe par gravité et est guidée par un entonnoir vers un conteneur ou un moyen de transfert, p.ex. une bande transporteuse. Ces mouvements non contrôlés occasionnent la perte de l'orientation de l'alvéole par rapport à la machine et rendent nécessaire pour les opérations suivantes une préhension et un réalignement de l'alvéole par rapport à une référence liée avec à cette prochaine opération.

La demande WO2010095102 (A1) est un exemple selon lequel les plaquettes sont orientées et positionnées afin que le découpage se fasse bien dans les sillons étroits séparant les alvéoles. Après découpage, les alvéoles tombent aléatoirement sur une bande transporteuse pour être amenées dans un goulot d'alignement afin de les orienter convenablement pour leur insertion dans l'étape suivante (l'emballage, à l'occasion). Les systèmes découpant les alvéoles et les laissant tomber de façon aléatoire, ajoutent encore une dimension de complexité à leur récupération ultérieure, car non seulement leur orientation et leur ordre de succession sont perdues, mais aussi peuvent-elles se trouver renversées sur la tête.

Le découpage proposé par l'invention, par un couteau en L ou en T présente l'avantage d'une bonne productivité tout en maintenant, au moins partiellement, la position de l'alvéole, entourée pour une partie par les jambes du couteau en L ou en T. Toutefois, pendant le découpage même, des forces relativement importantes sont exercées sur la plaquette par le couteau. Ces forces tendent à déplacer la plaquette dans plusieurs sens : elle sera globalement chassée hors de l'angle de cisaillement pendant la tranche du couteau. Il faut donc aussi bien tenir l'alvéole qui va en être séparée que la plaquette-même. Dans le système proposé, la plaquette est tenue par l'agrafe qui sert de référence de la position des alvéoles ; cette agrafe doit conserver cette position relative, toute au long du processus de découpage.

Le découpage même par couteau en L peut se faire de deux façons : en partant du bord de la plaquette vers l'intérieur avec des entailles qui se rencontrent et se croisent à l'angle-talon du L ou bien en commençant par un piquage (120) au talon du L, pour progresser vers les bords de la plaquette. Ce piquage peut être assuré par une pointe, prévue au talon du couteau en L. De la même façon, le piquage peut être assuré par une pointe, situé proche des coins du T (c.a.d. les coins entre la partie horizontale du T et la partie verticale). Ce découpage est effectué tandis que la plaquette se trouve sur une enclume .

Lors d'un découpage commençant par le poinçonnage au talon du L, cette partie du couteau ancre la plaquette ainsi que l'alvéole, jusqu'au moment ou les entailles débordent sur l'extérieur de la plaquette. Dans le cas général d'un découpage d'une alvéole, qui n'a pas une forme carrée, les entailles ne déboucheront pas toutes deux en même temps. Afin de contenir ces réactions sur l'alvéole, elle doit être fermement tenue, pendant le découpage. Ceci peut être obtenu par une pression sur la surface de l'alvéole, la plaquant sur l'enclume qui fait office de contre- lame et autour de laquelle cisaillent les lames du couteau en L. Cette pression axiale, dans le sens du mouvement de coupe du couteau, devra être active avant la première attaque du talon du couteau et persister jusqu'après le retrait complet des lames hors de la surface de la plaquette. En se retirant, le couteau aura tendance à entrainer la plaquette et à soulever l'alvéole de l'enclume. Puisque la position de la plaquette est toujours retrouvable tant qu'elle est tenue par l'agrafe, il est moins important de retenir la position de la plaquette pendant le retrait du couteau. Toutefois, la situation de l'alvéole, maintenant complètement désolidarisée de la plaquette, est tout à fait différente.

Les moyens de pression pressant l'alvéole sur la surface (plane) de l'enclume, peuvent être relaxés, sans perte de sa position si, par aspiration à travers d'orifices) pratiquées dans la superficie de l'enclume, l'alvéole y reste accolée. La continuité ininterrompue de la dépression dans le conduit d'aspiration de l'enclume confirme la qualité de la fixation de l'alvéole. Les alvéoles à traiter ont des tailles et des formats très variés, depuis les plus menues ayant par exemple une empreinte découpée de seulement 10mm x 10mm aux plus grosses avec par exemple une empreinte de 35mm x 40mm. La *tenue par aspiration de ces différents formats pourrait se faire en appliquant une aspiration sur toute la surface de l'enclume à travers des perforations.* Le débit aspiré est fonction de la superficie de l'enclume non couverte par l'alvéole, préjudiciant gravement la force d'aspiration pour les plus petites alvéoles. Etant donné les forces plutôt faibles obtenables par aspiration et le rendement mauvais des systèmes de pompe à vide, il est intéressant de moduler l'aspiration en fonction de la taille de l'alvéole à tenir. La prévision de plusieurs constellations de perforations connectées chacune par une vanne à son conduit vers le système de vide, permet de ne mettre en aspiration que les perforations couvertes par l'alvéole en découpage. Le résultat est une forte économie d'énergie et aussi la possibilité, même avec un seul système de vide commun, d'obtenir une forte aspiration de la pièce. L'application de ce système est rendu possible par la connaissance au préalable de la taille des découpages qui seront pratiqués sur les plaquettes. Même quand la taille varie en fonction de la position dans la plaquette de l'alvéole à découper, il sera possible de commander correctement la connexion des orifices d'aspirations couvertes par l'alvéole. De telle façon, une zone en dépression est créée agissant sur la superficie de l'alvéole découpée et, individuellement, ne dépassant pas cette superficie

Il a été constaté que typiquement, l'alvéole la plus rapprochée du point de fixation de l'agrafe, nécessite une manipulation spécifique. D'une part souvent l'agrafe gêne le positionnement entier de cette alvéole sur l'enclume de découpage et d'autre part la dernière alvéole peut avoir des dimensions qui diffèrent des dimensions des autres alvéoles. Puisque cette dernière alvéole ne peut, en certains cas, être séparée de l'agrafe par le couteau, il est prévu de pouvoir ouvrir l'agrafe, une fois que cettedernière alvéole est positionnée sur l'enclume et tenue en position par des moyens l'appuyant dessus.

Cette ouverture libère ce reste de la plaquette pour les transformations qui suivent.

Dans le cas le plus général, la forme de l'alvéole peut varier selon sa position dans la plaquette et il peut être nécessaire d'en tenir compte pendant les différentes opérations sur les alvéoles.

Les forces d'aspiration ainsi obtenues sont suffisantes pour conserver la position de l'alvéole relative à l'enclume pendant un déplacement tel que vers un prochain poste de travail.

La continuité ininterrompue de la dépression dans le conduit d'aspiration de l'enclume pendant un éventuel déplacement confirme la présence continue de l'alvéole.

Avec le système décrit, l'alvéole a conservé encore toujours son orientation, qui était connue avant le découpage par rapport à l'enclume.

Pour autant que tout transfert vers une opération suivante ne perturbe pas la position, les pertes de temps et les frais associés à un réalignement auront été évités.

Déplacement de l'alvéole tout en maintenant son orientation.(Fig. 7) Le déplacement de l'alvéole de son assise sur l'enclume, en conservant la connaissance d'orientation et positionnement relative à l'appareil, est nécessaire pour le déroulement ultérieur avec une meilleure accessibilité et afin de dégager l'endroit de découpage pour préparer la prochaine alvéole.

La préhension de l'alvéole accolée par aspiration peut se faire par tout moyen n'exerçant pas de forces qui perturberaient la position pendant la préhension et qui sont capables à surmonter la faible tenue par aspiration

Un choix approprié est la tenue par une ventouse aspirante (130), se conformant par ses lèvres souples (131) facilement aux différentes formes de la coupole de l'alvéole et par un soufflet (132), permettant une approche et un contact initial quasiment sans force. La relaxation de l'aspiration coté enclume permet au soufflet de la ventouse de soulever l'alvéole de l'enclume, tout en la dégageant pour un déplacement sans encombre. Tant que la dépression dans le soufflet de la ventouse n'a pas démarrée, la relaxation de l'aspiration au niveau de l'enclume est à éviter car cela pourrait entrainer la perte de la position et de l'orientation, conservée précieusement jusqu'à ce moment.

Avec cette solution, la tête ou cloche de l'alvéole se trouve encombrée par la ventouse, mais la surface plane arrière, généralement en feuillard d'aluminium est complètement exposée et accessible pour les opérations qui suivent.

Contrôle de la présence correcte et continue de l'alvéole pendant le déplacement relatif.

Le temps de cycle de l'appareil étant à raccourcir autant que possible, puisque ce temps est directement lié à sa productivité, les déplacements seront faits aussi rapidement que possible, impliquant des accélérations fortes sollicitant la tenue de la ventouse. Cette tenue par aspiration de l'alvéole peut être maximalisée, sans pour autant devoir changer de ventouse en fonction du type d'alvéole à prendre, par le choix d'une matière très souple avec une cloche d'ouverture importante par rapport au diamètre intérieur (133) du soufflet et de la canalisation d'aspiration. La taille maximale de cette cloche ne doit pas l'empêcher de se conformer autour des plus petites coupoles d'alvéoles à traiter, toute en restant suffisamment puissante pour tenir les plus grosses et lourdes alvéoles. Le compromis est finalement de choisir une ventouse soufflet capable de conformer aux plus petites alvéoles et le cas échéant, limiter les accélérations imposées lorsqu'il se produit des manque de tenues dues à la masse de l'alvéole ou à sa géométrie particulière. Les essais ont démontrés qu'un diamètre de 8mm de cloche de ventouse avec un passage intérieur par travers trois soufflets et demis en élastomère silicone, tel que fabriqué par Novacom sous la référence 8108-A, avec une dépression d'environ 0.5 atmosphère, faisait très bien l'affaire pour toutes alvéoles répertoriées.

Toutefois, le risque étant réel qu'une alvéole ne soit pas prise correctement ou soit perdue en route pendant le déplacement, un contrôle de sa présence avant la prochaine opération peut être nécessaire.

La mesure de la dépression dans le conduit reliant la ventouse à la pompe à vide donne une indication s'il y a bonne qualité de conformation de la ventouse à (la surface de) l'alvéole.

La continuité ininterrompue de la dépression dans la ventouse confirme la présence continue de l'alvéole.

Le mouvement de l'alvéole vers le prochain poste de travail et l'exécution de la prochaine étape de travail sont modifiés si une perte de position ou d'orientation est soupçonnée.

La modification peut être du genre interruption ou/et annulation de l'opération

Marquage du support d'emballage d'un identifiant concernant l'alvéole. La provenance de l'alvéole, son « identité personnelle », est toujours connue sans équivoque puisque l'alvéole a été tenue isolée depuis sa séparation de la plaquette et puisqu'il n'y a aucune autre alvéole séparée, avec laquelle la première alvéole pourrait être confondue.

Le maintien positif sans interruption de la tenue de l'alvéole, initialement indirectement dans la plaquette et après la séparation, par la tenue directe de l'alvéole a préservé toute information concernant position, forme et orientation à partir de la fixation de l'agrafe à la plaquette et l'attribution de son identifiant unique. Ceci permet la certitude de l'identité de l'alvéole lors de son transfert vers une position désirée sur un support d'emballage.

La préparation de cet emballage, qui est un emballage individualisé, n'est entamé qu'après confirmation que l'alvéole est correctement disponible à l'emballage. Un certain nombre de critères peuvent être utilisés pour établir cette confirmation, comme le niveau du vide d'aspiration dans la ventouse portant l'alvéole, le fait que l'alvéole a été soulevée de la surface de l'enclume, un signal de détection donné par des senseurs (vision, capteurs capacitifs...) de la présence de l'alvéole. Le respect de cette suite d'étapes élimine les risque d'erreurs en évitant la production d'un emballage « identifié » auquel pourrait ne pas être associé d'alvéole du tout ou, pire encore, pourrait être associé une alvéole non-correspondante.

Différents supports d'emballage connus peuvent servir, chaque fois portant une alvéole à l'unité accompagnée des informations identifiant le médicament dans l'alvéole., Tous ces systèmes démontrent l'importance pour avoir sur le support transportant le médicament des caractéristiques du médicament afin d'éviter confusion et pour rendre possible l'historique d'un médicament individuel. Toutefois, ces systèmes présentent des emballages de médicaments, qui sont passés par au moins une étape ou les alvéoles étaient mélangées avec d'autres dans le désordre, avant d'être individualisées par une identification. Ils n'ont donc pas la possibilité de présenter, sans le moindre doute, sur le support les informations qui se trouvaient sur l'emballage secondaire (qui sont considérées être le plus fiables), ni la possibilité de savoir de laquelle des plaquettes dans cet emballage secondaire (il peut y en avoir 5, 15 ou plus dans une boite) est issue l'alvéole, ni dans quelles conditions, ni à quel instant la plaquette ou les alvéoles qu'elle portait ont été découpées, ni par quel appareil, surveillé par quel opérateur. Autant d'éléments utiles au suivi d'une automation se voulant traçable et fiable pour le traitement de matières aussi délicates que les médicaments.

Le système selon l'invention, n'ayant à aucun moment perdu l'identité du médicament , ni d'une alvéole individuelle, propose de matérialiser sans équivoque les informations concernant le médicament sur le support auquel est fixé indissociablement l'alvéole et propose un moyen de le faire. L'invention propose d'utiliser une carte réunissant l'alvéole avec des informations lisibles à l'oeil ainsi que d'autres informations lisibles par des automates.

### Choix du système de marquage

Comme défini plus haut, une identification sans équivoque doit être associée à l'alvéole lors de sa fixation à son support d'emballage: cela présuppose que le marquage de l'identification sur le support d'emballage soit indissociable de celui-ci et n'y soit apporté que pendant la période de temps ou seule l'alvéole correspondante est séparée de la plaquette.

Plusieurs systèmes on été proposés pour apporter le marquage sur le support d'emballage tels que des systèmes à laser, l'impression par thermotransfert, l'impression par sublimation, l'impression sur papier thermique ou l'impression par jets d'encre.

Dans le cadre de l'invention, le système HP de DOD (drop-on-demand) par jets thermiques est préféré au jet piézo-électriques car ce système est très compacte, contenant son réservoir d'encre ainsi que de l'électronique embarquée et est aussi économique (remplacement intégral après consommation de l'encre).

On peut encore mentionner les *étiquettes précollées, qui* peuvent servir pour autant que leur adhérence au support d'emballage soit permanente.

Fixation de l'alvéole sur le support d'emballage. L'alvéole peut être fixée sur le support d'emballage par différents moyens, parmi lesquels on peut citer: un agrafage mécanique de l'alvéole, une bande adhésive , la fusion par LASER, un système de passepartout emprisonnant l'alvéole ou la fixation permanente de l'alvéolé à son support d'emballage par un adhésif.

Plusieurs possibilités de fixation appartiennent à cette dernière catégorie : par exemple le hot-melt, la colle à froid, le heat-seal etc décrits dans les demandes EP1800645 (A1), WO2008135825 (A2), NL1033807 (C2), GB1507828 (A) heat seal, US3307693 (A), WO2010095102 (A1) ethilog, US3942640 (A). Ces systèmes ont tous leur spécificités.

Le hot-melt travaille à des températures avoisinantes celles de fusion de la matière des cloches d'alvéoles, mais peut être avantageusement déposé du coté feuillard plan, plus éloigné de la cloche et en général fait d'une matière plus épaisse et/ou thermiquement plus résistante, comme l'aluminium.

Une chaleur excessive apportée par la colle hot-melt provoquerait des dégâts à l'alvéole ou au médicament, ce qui proscrit l'application de grosses masses de colle par rubans ou filins typiquement utilisée. Toutefois en dosant le hot-melt en petites gouttelettes d'environs 0.01 cm3 à 0.02cm3 la chaleur apportée est réduite sous le seuil de danger. La projection de telles infimes quantités de colle à chaud au moment voulu et avec la précision nécessaire pour que la colle se trouve proche des bords d'une alvéole, demande un appareil de projection fin. Le choix est ouvert entre manipuler le pistolet à colle ou manipuler l'alvéole: leur mouvement relatif est nécessaire afin de positionner correctement les gouttelettes sur toute la variété d'alvéoles à traiter. Une solution avantageuse consiste à se servir du déplacement de l'alvéole depuis la table de découpage vers le poste de fixation au support d'emballage pour passer l'alvéole devant un pistolet à colle fixe dans l'espace. Les pistolets à colle hot-melt projettent la colle par une buse dans laquelle est incorporée la vanne, le tout monté sur un corps formant réservoir sous pression et tenu au-delà de la température de fusion de la colle. Ce corps chaud est tenu à environ 150°C et émet par radiance une chaleur considérable, qu'il est judicieux de tenir à l'écart de l'alvéole. La projection des gouttelettes mentionnées sur une distance de 50mm à 100mm est stable et répétitive et permet de satisfaire à l'écartement physique et séparation thermique du pistolet du chemin de passage de l'alvéole et tous les éléments associés. Le besoin d'une cadence élevée exige une vannerie à haute réactivité et pilotée par un processeur en synchronisation étroite avec le manipulateur.

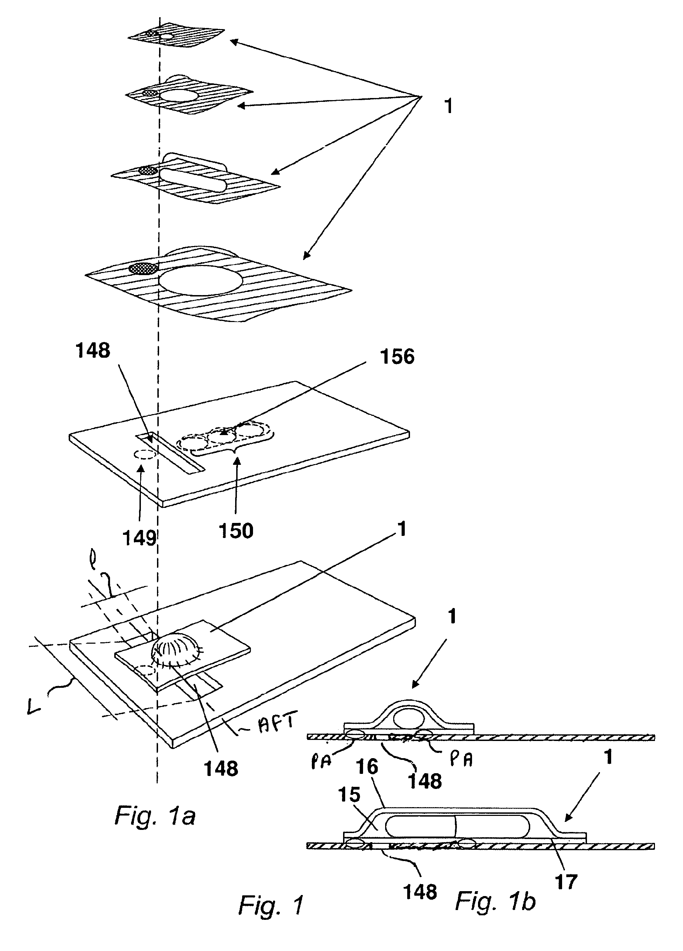

### Choix de forme de supports d'emballage pour faciliter l'expulsion ultérieure du comprimé (Fig. 1a & 1b)

Un des buts du système est de le rendre plus facile à utiliser par le personnel, chargé de contrôler la correspondance entre les médicaments dispensés et ceux à administrer à un patient après l'ordonnance du patient, de contrôler le conditionnement correcte des médicaments (date de péremption, dosage) et aussi chargé de l'administration des médicaments comprenant le déconditionnement (extraction de l'emballage primaire) et l'enregistrement éventuels de ces opérations. L'objectif de rendre lisible et accessible les informations concernant les médicaments est déjà atteint par l'impression des identifiants et autres données sur les supports d'emballage.

Pour la fixation indissociable de l'alvéole à la surface de son support d'emballage, un point de colle suffirait, mais la récupération de la pilule se trouvant sous la cloche de l'alvéole serait rendue difficile.

Selon un mode de réalisation avantageux de l'invention, une solution est proposée pour récupérer la pilule. Cette solution rend facile et plus sûr la rupture de l'opercule par un mouvement standardisé pour toutes les formes et tailles d'alvéoles. Il est connu en ingénierie qu'une concentration de stress (« stress-concentration » en anglais) multiplie invisiblement les forces mécaniques et provoque la rupture des matières: cet effet est mis en oeuvre pour amorcer la déchirure de l'opercule de l'alvéole à un coté de l'alvéole ou à une extrémité dans le cas d'alvéoles non-circulaires.

Le principe de l'invention est de prévoir une ouverture de faiblesse (148) (également dénommée fente de faiblesse) dans la carte située sous l'alvéole. Cette ouverture n'est pas fonctionnellement qu'une entaille ou fente formée, mais permet de réaliser une faiblesse orientée au travers d'un bord de l'alvéole et a une longueur dépassant les bords de l'alvéole (Fig. 1a).

Comme illustré sur la figure 1a, la fente de faiblesse 148 est disposée transversalement sur la carte et confère un axe de faiblesse transversal AFT à la carte autour duquel la carte peut se rompre. Les points d'attache PA (du tambour pelliculaire de l'alvéole contenant le comprimé) sur la carte sont situés de part et d'autre de la fente de faiblesse 148 (Cf. figure 1b). La fente de faiblesse 148 présente une ouverture minimale évitant que les points d'attache PA ne puissent obstruer la fente. Pour les comprimés trouvés dans le commerce, une ouverture présentant une longueur L de l'ordre de 15 à 20 mm suffit pour les laisser passer. La fente de faiblesse est disposée en travers de l'alvéole et un bord du tambour de l'alvéole est tangent à un bord de la fente de faiblesse.

Ces caractéristiques permettent d'obtenir une surtension dans l'opercule obturant l'alvéole lorsque la carte est craquée suivant l'axe de faiblesse AFT et provoquant l'amorce de rupture à un endroit précis et voulu. On obtient ainsi une ouverture large pour le passage du comprimé.

Il convient par ailleurs de noter que la surface de la fente de faiblesse est inférieure à la surface de l'opercule. La fente de faiblesse change complètement de physiologie lorsque la carte est craquée le long de l'axe de faiblesse AFT. En effet, un bâillement important se créé au niveau de la fente de faiblesse et permet d'extraire facilement le comprimé.

L'invention prévoit que l'opercule est fixée (par collage) préférentiellement à au moins deux endroits (149,150) répartis de part et d'autre de la faiblesse. En différence avec les emballages connus munis d'un point de faiblesse dans la carte, ce mode de réalisation avantageux prévoit que l'opercule (de l'alvéole à ouvrir) est fixée (par collage) à au moins deux endroits répartis de part et d'autre d'un point de faiblesse. Avec cette disposition, une pression sur le sommet de la cloche de l'alvéole (par un pouce) alors que la carte est tenue fermement entre pouce et index de l'autre main du coté où se trouve la faiblesse et le bord de l'alvéole, provoque directement la rupture de l'opercule au bord de l'alvéole et son déchirement en circonférence, pendant que le comprimé se trouve éjecté à travers la carte par la fente. En fin de mouvement la carte est repliée en forme de U avec l'alvéole éventrée dans le creux du U et la fente ouverte au bas. Pour le bon fonctionnement du principe, le positionnement relatif des points de fixation de l'opercule à la carte fendue est important et n'est pas évident. Comme déjà décrit les points de fixation se trouvent de part et d'autre de la fente. Etant donné que la fente est transversale, ces points de fixation sont sensiblement alignés dans la longueur de la carte.

Puisque l'alvéole est placée avec la fente tangente à la circonférence de la cloche (en réalité tangente à la projection de la circonférence de la cloche sur la carte), l'alvéole est disposée fort asymétriquement par rapport à la fente et un coté ne dépasse la fente que de peu. Cette partie, qui est une partie ou l'opercule est fusionnée avec la matière formant la cloche, est relativement rigide. Un point de colle fixera cette partie rigide d'un coté de la fente. De l'autre coté de la fente se trouve essentiellement la partie de l'alvéole recouverte par l'opercule, comme une peau de tambour. Le second point de colle est planté vers le milieu du « tambour » formé par l'opercule couvrant la cloche de l'alvéole. L'espacement de ce point de colle avec le premier est donc de plusieurs millimètres, même pour les plus petites alvéoles. Lorsque la carte est manipulée comme décrit plus haut, la pression pousse le comprimé vers l'opercule du coté de l'alvéole le plus proche de la fente et l'opercule, tenu par son milieu se trouve d'autant plus tendue par le soutient de la carte qui commence à bailler au niveau de la fente de faiblesse. Le manque de support de l'opercule à cet endroit et le surcroit de pression du comprimé vers cet emplacement provoque la concentration d'efforts (stress concentration) recherché qui dépasse la résistance de l'opercule. Une amorce de rupture se forme du coté de l'alvéole tangent à la fente de faiblesse et progresse, guidée par le repli de la carte en forme de U, ouvrant d'autant plus la fente pour laisser le passage libre au comprimé pour sortir. Nous l'avons déjà vu, les alvéoles ont des tailles et des formes et matières diverses : avec ce système, un seul type de mouvement d'ouverture suffira pour expulser toutes les variantes, car il est toujours possible pour le constructeur de l'appareil de maintenir la disposition des deux points de colle ainsi que l'emplacement précis de l'alvéole par rapport à la fente de faiblesse de la carte pour obtenir le résultat escompté. Typiquement, il s'avère qu'il n'est pas nécessaire de choisir le milieu de la peau de tambour pour le deuxième point de colle pour obtenir le bon résultat sur des grosses alvéoles et qu'une position intermédiaire (156) entre le premier point de colle et le milieu est optimal.

En réalité, les points de colle ne sont pas des points mes des plots de colle ayant une dimension finie lorsque l'alvéole encollée est appliquée à la carte.

Quand l'alvéole est placée avec le bord du tambour tangent à la fente de faiblesse pour obtenir les effets de concentration de stress, le plot de colle placé en bord rigide du tambour de l'alvéole risque, en s'aplatissant, de couler et colmater une simple entaille faisant office de fente dans la carte. Cela empêcherait le bon fonctionnement du système car la fente s'en trouverait « renforcée » par la colle. Pour éviter le débordement sur la fente du plot de colle aplati, il est possible de reculer son centre de quelques millimètres. Toutefois, cela est contraire au bon fonctionnement, car cela implique de reculer aussi l'alvéole et donc que le bord du tambour ne soit plus tangent à la fente de faiblesse. Pour éviter avec certitude que ce premier plot de colle ne puisse ponter la fente et la colmater, il est avantageux que la fente présente une largeur 1 d'environ 3 mm. La longueur L de la fente peut par exemple être comprise entre 10 et 15 mm.

Le premier et le deuxième plots de colle ne doivent non plus se rencontrer et former un pont résistant « renforçant » la fente de faiblesse. Ceci est également assuré par l'ouverture de 3 mm de la fente. Donc, même avec une alvéole de petite taille dont les deux points de fixation sont nécessairement rapprochés, le pontage par la colle de la fente est évité.

L'invention prévoit donc au moins deux points de fixation de l'alvéole à la carte séparés par la fente de faiblesse.

### Positionnement des plots de colle; Poste d'encollage

Le choix existe entre encoller l'alvéole et puis l'appliquer sur la carte ou l'inverse. Des essais industriels ont démontrés que la colle à chaud a une bien meilleure tenue si elle est d'abord appliquée à la surface avec la plus grande capacité d'évacuation de chaleur et ensuite à la surface plus isolante. Dans le contexte présent il s'agit d'appliquer la colle chaude d'abord à l'opercule aluminées de l'alvéole et ensuite au cartonnage de la carte, support d'emballage.

Comme décrit plus haut, l'un des points de colle sera posé en dehors du bord du tambour de l'alvéole et l'autre vers son centre. Pour éviter de provoquer des dégâts au médicament derrière l'opercule par conduction de chaleur le plot de colle est dosé en quantité inférieure à 0.02cm3. La chaleur apportée par la gouttelette de hot-melt, liquide à une températures au-delà de 110°C, provoque une montée en température inférieure à celle subie lors de l'emballage primaire du comprimé dans le blister dans une ligne de production.

La projection de colle à chaud avec la précision pour un espacement de quelques millimètres seulement entre plots est réalisée en déplaçant l'alvéole au ralenti devant le pistolet à colle. Les deux gouttelettes, projetées avec une vitesse de plusieurs m/sec, atteignent l'alvéole d'une façon régulière espacée de quelques millisecondes dans le temps. Certains supports combinés avec certaines colles donnent une meilleur adhérence s'il y a un temps de repos pelliculant la colle entre son application au premier support et le collage définitif. Aussi, il est choisi de placer d'abord le plot de colle ayant le plus long parcours thermique, celui du milieu de l'alvéole, et celui en bordure en second ayant une plus haute conduction thermique par travers la structure de l'alvéole. Pour l'application en question sur des alvéoles et avec les tailles de plots de colle choisis suffisamment petits, le temps de repos ne dépasse pas le temps nécessaire à déplacer l'alvéole encollé vers le support d'emballage pour son affixation définitive et une productivité optimal peut etre atteinte.

Les aux moins deux gouttelettes pour fixer une alvéole peuvent être de tailles différentes afin d'obtenir une adhésion optimale

### Orientation de l'alvéole par rapport au support d'emballage.

En général, le découpage de l'alvéole n'est pas fait dans l'orientation voulue pour la fixation au support d'emballage.

Dans des cas connus de fixation de l'alvéole sur ou à travers une carte comme, il est important de maitriser l'orientation lors de la fixation de l'alvéole à la carte. Cette maitrise exige connaissance de l'orientation des deux éléments (ou du moins de leur orientation relative) plus la possibilité de faire au moins une rotation pour réduire l'écart avec l'orientation relative voulue. Une économie de rotations est obtenue si lors de leur fixation permanente les deux éléments de l'ensemble sont orientés correctement pour l'opération qui suivra.

### Provenance des cartes et implications

Les cartes utilisées dans le système peuvent provenir d'une bande continue ou d'une pile de cartes, sans incidence sur ce qui a été défini plus haut. Toutefois, le déroulement des opérations sera différentes selon le cas. Dans le cas ou les cartes sont issues d'une bande elles sont liées entre-elles. Donc toute manipulation des cartes influence l'ensemble des cartes en amont. Il est clair que tout mouvement des cartes dans un sens qui peut rendre une des opérations difficiles, doit être limité ou corrigé. Pendant les positionnements ou l'impression, la bande avec les cartes aura été tirée dans le sens de mouvement de la carte traitée. Il est clair que dans ce cas, des rotations de la carte sont difficilement intégrables et que les mouvements devraient se limiter à des transports dans le sens longitudinal de la bande, excluant aussi les mouvements latéraux. Ces restrictions n'empêchent pas l'exécution des taches décrites, elles font que dans certains cas, le choix n'est pas ouvert entre manipuler l'alvéole ou la carte. Dans la suite des étapes, l'alvéole, ayant subie la plupart des préhensions mécaniques, est le sujet préféré pour faire les positionnements et orientations. Avec un « train de cartes » dans une orientation fixe relative au bâti de l'appareil et un positionnement uni-axial, les positionnement et orientations de l'alvéole relatives au bâti le sont aussi relatives au train de cartes.

Dans le cas de cartes issues d'une pile, les mêmes conditions peuvent être obtenues que plus haut. Par contre, en modifiant pendant les étapes le positionnement latéral de la carte ou son orientation par rapport au bâti de l'appareil, ces grandeurs seront également modifiées par rapport à l'alvéole.

Orientation et Position relatives de l'agrafe, la plaquette, l'alvéole, la carte, le bâti de l'appareil.

Contraintes et Choix. Il a été vu que les agrafes sont fixées dans une position et orientation relative aux alvéoles des plaquettes.

L'ensemble agrafe et plaquette est formé séparément de l'appareil de transformation et peut être entreposé indépendamment : le choix d'orientation à l'introduction dans l'appareil de transformation facilitera les transferts.

Une fois présentée à l'appareil de transformation l'agrafe ne change plus d'orientation, donc la plaquette non plus.

Les alvéoles peuvent être disposées orientées correctement sur un espace biaxial : pour leur découpage deux déplacements latéraux sont à prévoir Une fois découpée de la plaquette, l'alvéole peut assumer la bonne orientation et position relative à la carte, pour achever la transformation.

Un train de cartes ne change ni de position latérale ni d'orientation pendant la transformation.

Donc , dans le cas d'un train de cartes, l'alvéole doit assumer la position latéralement par rapport aux train de carte et l'orientation de celui-ci.

Le cas le plus simple verrait le train de cartes perpendiculairement en-dessous de l'alvéole découpée, centré latéralement et orienté suivant l'orientation voulue des alvéoles sur les cartes.

Puisque le découpage des alvéoles nécessite deux axes de translation, le train de cartes, n'ayant qu'un axe de déplacement dans sa longueur, devrait se déplacer latéralement en entier pour rester à l'aplomb des alvéoles.

Pour des raisons de constructions, d'encombrements et d'imbrications d'éléments, il est préféré de séparer latéralement l'axe du train de cartes de la position de découpage.

L'orientation relative entre le train de cartes et les alvéoles est un choix libre, influencé par les moyens mis en oeuvre pour compenser les différences géométriques entres les type d'alvéoles et pour faire le déplacement latéral devenu nécessaire.

Pour des raisons de constructions, d'encombrements et d'imbrications d'éléments, il est préféré de fixer latéralement l'axe du train de cartes et d'y amener l'alvéole latéralement depuis la position de découpage.

Pour des raisons de constructions, d'encombrements et d'imbrications d'éléments, il est préféré de déplacer latéralement la position de découpage pour porter l'alvéole à une position intermédiaire par rapport au train de cartes.

Depuis la position intermédiaire les alvéoles peuvent être transportées latéralement vers l'axe du train de cartes.

Pour des raisons de propreté, de facilité de nettoyage et d'entretien, il est préféré de situer toute la mécanique de la machine au dessus du niveau de découpage et/ou du niveau d'encollage : les déchets et ratés tombent hors de la partie active.

Pour des raisons de propreté, il est préféré de positionner le pistolet à colle au point le plus bas du trajet des alvéoles : les éventuels gouttes de colle ratées ne tombent pas sur des parties actives de l'appareil.

Les imprimantes à jet d'encre ne fonctionnent qu'en projection horizontal ou inclinée vers le bas, mais pas vers le haut.

Du fait de ces choix, il est avantageux que le train de carte va de haut en bas, avec les cartes situées selon la verticale.

Les imprimantes à jet d'encre peuvent être très proche (1 mm) du support à imprimer, donc l'impression d'une carte avec une alvéole fixée dessus n'est plus possible du coté alvéole.

Les deux cotés de la carte peuvent porter l'identifiant du médicament.

Il s'ensuit que les imprimantes se trouvent en amont de l'assemblage.

Chaque assemblage de carte, alvéole et impression d'identifiant étant terminé avant d'entamer un prochain cycle le train reculera en amont de la première tête d'imprimante puis repassera devant en descendant pour la prochain carte.

La séparation par massicot de la dernière carte assemblée peut se faire sur le trajet de rétraction et épargner aussi bien des distances de mouvement supplémentaires qu'un massicot ayant un gueule assez large pour avaler les alvéoles.

La position d'assemblage doit être sensiblement à la même hauteur que celle du découpage.

Pour des raisons de précision par séparations des axes et puisque le découpage est mobile latéralement, le second axe de la position de découpage déplace l'agrafe et la plaquette le long de la verticale sans translation latérale.

Le découpage se fait toujours à la même hauteur.

Le position verticale du centre de l'alvéole découpée varie en fonction de la dimension verticale de l'alvéole et la forme de la cloche.

La transposition latérale de l'alvéole vers la position d'assemblage au train de cartes doit tenir compte de la position latérale du centre de l'alvéole découpée.

La préhension fiable de diverses formes d'alvéoles par une ventouse générique, oblige le positionnement de l'alvéole sous la ventouse, approximant à un centrage .

Le centrage oblige un positionnement relatif selon deux axes (vertical et latéral).

La compensation selon l'axe latéral peut être réalisée par le transport latéral depuis la position de découpage à la position intermédiaire (qui est désormais variable).

La compensation selon l'axe vertical est partiellement réalisée par le positionnement plus ou moins haut ou bas du train de carte lors de la fixation de l'alvéole.

La compensation du centrage selon l'axe vertical pour la prise de l'alvéole est combiné avec le passage devant le pistolet à colle et avec le déplacement latéral en un seul mouvement rotatif.

Un mouvement rotatif passe l'alvéole le long d'une trajectoire horizontale devant le pistolet à colle : les coulures de colle des deux plots ne se mélangeront pas, l'encollage est le point le plus bas de la trajectoire.

La compensation rotative introduit une inversion de l'alvéole pendant son déplacement vers le train de cartes, sans incidence sur le système.

La compensation selon l'axe verticale par bras rotatif, engendre un petit défaut de positionnement latéral lors de la pose de l'alvéole sur la carte, dont le train est à distance fixe du pivot du bras.

L'orientation inversée de l'alvéole sur la carte mets ses bords exactement parallèles à la carte, pour autant que le bras balaie toujours le même angle de rotation entre la préhension et la fixation.

La distance de rétraction vers le massicot de séparation de la carte portant l'alvéole nouvellement fixée est variable suivant les compensations.

La distance de rétraction de train de cartes est fixe depuis le massicot pour réinitialiser l'imprimante.

La carte séparée par le massicot à une position et orientation fixe dans l'espace.

La séparation de la carte individuellement identifiée avec l'alvéole indissociablement affixée clôture le cycle de transformation et libère l'appareil pour la préparation de la prochaine carte.

La préhension et le transport de cette carte vers un entreposage organisé peut être automatisé : pince, piston éjecteur.

L'axe préféré pour le mouvement d'éjection est perpendiculaire au deux axes de translation mentionné ci-dessus : la carte terminée sort du plan de travail de l'appareil de transformation et de l'emprise du massicot.

La conservation de l'ordre de production des cartes pendant le stockage est assuré en les y enfilant à la queue-leu-leu.

L'enfilade peut être assurée par un rail, un anneau ou un canal, compartimenté par un pas de vis ou des parois moulées.

L'ensemble carte, identifiants, alvéole étant créé, a une position et une orientation qu'il est utile de conserver pour les manipulations ultérieures.

### Fonctionnement du système de transformation

Les blisters montés sur des valets, pinces " ou clip " ou encore agrafes peuvent être chargés par l'opérateur sur un carrousel.

Les pinces peuvent être munies d'un système RFID et la machine d'un lecteur permettant de savoir ce qui est monté dans la pince

Le carrousel pourra contenir une vingtaine de blisters

L'orientation des blisters sur les clips peut être faite sur un poste spécifique

L'accès au carrousel peut être protégé par une barrière immatérielle qui bloquera les mouvements dangereux.

Un portique à deux axes numériques muni d'un système de préhension de valets peut saisir les blisters dans le carrousel

Un système de couloir matérialisé par une plaque sur l'avant de la machine permettra de redresser les blisters courbes.

Cette plaque commencera par un avaloir pour facilité le passage des blisters dans le couloir.

Cette plaque devra être mobile pour échapper à l'ensemble des mouvements du reste de la machine.

Un second axe numérique X munie d'un système de couteaux et d'enclume découpage les blisters selon une procédure contenue dans un fichier.

Dans la majorité des cas la dernière alvéole ne pourra être séparée de la pince. Il faut donc prévoir un système permettant d'ouvrir le clip pour libérer l'alvéole. L'enclume peut être munie d'une série de trous permettant l'aspiration et le maintient de l'alvéole.

En fonction de la taille de l'alvéole certains trous d'aspiration peuvent être activés: de préférence 3 zones d'aspiration.

Les couteau peuvent être en forme de L avec un ergot dépassant une des jambes du L.

Les couteaux peuvent être déplacés par un vérin.

L'enclume et les couteaux peuvent être mis en référence par une même pièce.

Un bloc de matière visco-élastique prisonnier entre les couteaux peut aplatir les blisters courbe avant le découpage

Un système de bras rotatif munie d'une ventouse peut saisir l'alvéole découpée.

La rotation peut être faite par un moteur électrique

Pendant la rotation l'alvéole est présentée devant une buse de colle qui déclenche deux fois un jet en fonction des infos codeur du moteur.

L'alvéole peut être présentée devant la carte pour y être appliquée.

Les alvéoles peuvent avoir une épaisseur allant de 1.5 à 10.5 mm. La compensation de cette variation sera faite par une table numérique sur laquelle sera embarqué le moteur du mouvement de rotation ou la ventouse sera monté au bout d'un actionneur.

Le positionnement final de l'alvéole sur la carte est primordiale.

Le système selon l'invention et la méthode selon l'invention comprennent chacun une pluralité de caractéristiques, décrites en tant que caractéristique de système ou en tant que caractéristique de méthode. Il est entendu qu'à chaque caractéristique de système correspond une caractéristique de méthode. De plus, la description présente les différentes caractéristiques dans une certaine combinaison. Il est entendu que les combinaisons décrites ne sont pas limitatives pour l'invention mais que l'invention comprend aussi d'autres possibilités de combinaison de ces caractéristiques, sans sortir du cadre de la présente invention telle que définie dans les revendications en annexe.

## Revendications

1. Système automatisé (1) apte à la délivrance sécurisée d'une dose unitaire d'un médicament à partir d'une plaquette comprenant des alvéoles contenant le médicament, le système comprenant :
- des moyens pour fixer une agrafe sur la plaquette ;
- des moyens pour positionner et orienter cette agrafe par rapport à la plaquette en fonction du positionnement et de l'orientation des alvéoles et/ou des sillons séparant les alvéoles sur la plaquette ; et
- des moyens pour enregistrer :
o des données concernant le positionnement et l'orientation des alvéoles sur la plaquette,
o des données concernant le positionnement et l'orientation des sillons séparant les alvéoles sur la plaquette, ainsi que
o des données concernant l'identification de la plaquette ainsi que des données concernant l'identité, position et orientation de l'agrafe attachée à la plaquette.

2. Le système automatisé de la revendication 1 dans lequel l'agrafe est orientée selon l'axe d'un des sillons séparant les alvéoles et/ou positionnée en deux dimensions, la position en deux dimensions étant définie par un agencement suivant les caractéristiques géométriques de la plaquette d'alvéoles.

3. Le système automatisé de la revendication 1 ou 2, dans lequel l'agrafe comprend des moyens pour enregistrer des données concernant le positionnement et/ou l'orientation des alvéoles sur la plaquette et/ou des données concernant le positionnement et l'orientation des sillons séparant les alvéoles sur la plaquette et/ou des données concernant l'identification de la plaquette.

4. Le système automatisé de la revendication 1 à 3, qui comprend en plus :
- des moyens de découpage d'au moins une alvéole de ladite plaquette qui sont pilotés par les données concernant le positionnement et/ou l'orientation des alvéoles sur la plaquette et/ou par les données concernant l'identification de la plaquette, et/ou
- qui comprend en plus des moyens de traitement des alvéoles, prélevées de la plaquette qui sont pilotés par les données concernant le positionnement et/ou l'orientation des alvéoles sur la plaquette et/ou par les données concernant l'identification de la plaquette.

5. Le système automatisé selon la revendication 4, dans lequel la plaquette et les moyens de découpage sont positionnés l'un par rapport à l'autre suivant les données enregistrées dans l'agrafe.

6. Le système automatisé de la revendication 1 à 5 muni de moyens pour libérer la plaquette de l'agrafe.

7. Le système automatisé selon une des revendications 4 à 6, dans lequel les moyens de traitement des alvéoles, prélevées de la plaquette comprennent des moyens pour maintenir la position et l'orientation de l'alvéole pendant et après le découpage.

8. Le système automatisé selon une des revendications 4 à 7, qui comprend en plus des moyens pour déplacer l'alvéole découpée en conservant l'information concernant la position et l'orientation de l'alvéole telle que découpée.

9. Le système automatisé selon la revendication 8 qui comprend en plus un poste d'encollage et dans lequel les moyens de déplacement positionnent l'alvéole devant le poste d'encollage pendant le trajet vers la position de fixation sur la carte.

10. Le système automatisé selon une des revendications 1 à 9, qui comprend des moyens pour apporter des informations identifiant le médicament sur la carte après le découpage de l'alvéole contenant le médicament.

11. Le système automatisé selon une des revendications 1 à 10 comprenant des moyens pour ranger les doses unitaires une à une, dans l'ordre de leur production, dans un étui prévu à cet effet.

12. Méthode pour la délivrance sécurisée d'une dose unitaire d'un médicament à partir d'une plaquette comprenant au moins une alvéole contenant le médicament, la méthode comprenant les pas suivants :
- la fixation d'une agrafe sur la plaquette ;
- la détermination de la position et de l'orientation de cette agrafe par rapport à la plaquette en fonction du positionnement et de l'orientation des alvéoles et/ou des sillons séparant les alvéoles sur la plaquette;
- l'enregistrement :
∘ des données concernant le positionnement et l'orientation des alvéoles sur la plaquette,
∘ des données concernant le positionnement et l'orientation des sillons séparant les alvéoles sur la plaquette, ainsi que
∘ des données concernant l'identification de la plaquette ainsi que des données concernant l'identité, la position et l'orientation de l'agrafe attachée à la plaquette.

13. Méthode pour la délivrance sécurisée d'une dose unitaire d'un médicament à partir d'une plaquette selon la revendication 13, dans laquelle au moins une partie des données enregistrées est enregistrée sur l'agrafe.

14. Méthode pour la délivrance sécurisée d'une dose unitaire d'un médicament à partir d'une plaquette selon une des revendications 13 ou 14, qui comprend :
- l'orientation de la plaquette par rapport aux axes du couteau, utilisé pour découper l'alvéole de la plaquette;
- le découpage de l'alvéole de la plaquette.

15. Méthode pour la délivrance sécurisée d'une dose unitaire d'un médicament à partir d'une plaquette selon une des revendications 13 à 15, qui comprend la fixation de l'alvéole découpée sur une carte, cette fixation n'étant exécutée qu'après une confirmation qu'il n'y a pas de doute sur l'identité de l'alvéole à fixer.

## Patentansprüche

1. Automatisiertes System (1), das zur gesicherten Ausgabe einer Einzeldosis eines Medikaments ausgehend von einer Plakette mit der Vertiefungen, die das Medikament enthalten, geeignet ist, wobei das System umfasst:
- Mittel zur Befestigung einer Klammer auf der Plakette;
- Mittel zur Positionierung und Orientierung dieser Klammer im Verhältnis zur Plakette in Abhängigkeit von der Positionierung und der Ausrichtung der Klammern und / oder der Spuren, die die Vertiefungen auf der Plakette trennen; und
- Mittel zum Speichern:
o der Daten bezüglich der Positionierung und der Ausrichtung der Vertiefungen auf der Plakette,
o der Daten bezüglich der Positionierung und der Ausrichtung der Spuren, die die Vertiefungen auf der Plakette trennen, sowie
o der Daten bezüglich der Identifikation der Plakette sowie Daten hinsichtlich der Identität, Position und Ausrichtung der an der Plakette befestigten Klammer.

2. Das automatisierte System gemäß Anspruch 1, in dem die Klammer gemäß der Achse einer der Spuren ausgerichtet ist, die die Vertiefungen trennt, und / oder in zwei Dimensionen positioniert ist, wobei die Position in zwei Dimensionen durch eine Anordnung gemäß den geometrischen Merkmalen der Vertiefungsplakette definiert ist.

3. Das automatisierte System aus Anspruch 1 oder 2, in dem die Klammer Mittel zum Speichern der Daten hinsichtlich der Positionierung und / oder der Ausrichtung der Vertiefungen auf der Plakette und / oder Daten bezüglich der Positionierung und der Ausrichtung der Spuren, die die Vertiefungen auf der Plakette trennen, und / oder Daten bezüglich der Identifizierung der Plakette umfasst.

4. Das automatisierte System aus Anspruch 1 bis 3, das darüber hinaus umfasst:
- Zuschnittsmittel von wenigstens einer Vertiefung der genannten Plakette, die durch die Daten bezüglich der Positionierung und / oder der Ausrichtung der Vertiefungen auf der Plakette und / oder durch die Daten bezüglich der Identifizierung der Plakette gesteuert werden, und / oder
- das darüber hinaus Verarbeitungsmittel der Vertiefungen umfasst, die aus der Plakette entnommen werden, die durch Daten bezüglich der Positionierung und / oder der Ausrichtung der Vertiefungen auf der Plakette und /oder durch die Daten bezüglich der Identifizierung der Plakette gesteuert werden.

5. Das automatisierte System gemäß Anspruch 4, in dem die Plakette und die Zuschnittsmittel im Verhältnis zueinander gemäß den in der Klammer gespeicherten Daten positioniert sind.

6. Das automatisierte System aus Anspruch 1 bis 5, das mit Mitteln zur Freisetzung der Plakette von der Klammer ausgerüstet ist.

7. Das automatisierte System gemäß Anspruch 4 bis 6, in dem die Verarbeitungsmittel der Vertiefungen, die aus der Plakette mit Mitteln zur Aufrechterhaltung der Position und der Ausrichtung der Vertiefung während und nach dem Zuschnitt entnommen werden.

8. Das automatisierte System gemäß Anspruch 4 bis 7, das darüber hinaus Mittel zum Verschieben der zugeschnittenen Vertiefung unter Beibehaltung der Information hinsichtlich der Position und der Ausrichtung der Vertiefung in ihrem ausgeschnittenen Zustand umfasst.

9. Das automatisierte System gemäß Anspruch 8, das darüber hinaus einen Verklebungsposten umfasst und in dem die Verschiebungsmittel die Vertiefung während der Fahrt zur Befestigungsposition auf der Karte vor dem Verklebungsposten positionieren.

10. Das automatisierte System gemäß Anspruch 1 bis 9, das Mittel umfasst, um Informationen einzubringen, die das Medikament auf der Karte nach dem Zuschnitt der das Medikament enthaltenden Vertiefung identifiziert.

11. Das automatisierte System gemäß Anspruch 1 bis 10, umfassend Mittel zum einzelnen Verstauen der Einzeldosen in der Reihenfolge ihrer Produktion in einem zu diesem Zweck vorgesehenen Etui.

12. Verfahren für die gesicherte Ausgabe einer Einzeldosis eines Medikaments ausgehend von einer Plakette mit wenigstens einer das Medikament enthaltenden Vertiefung, wobei das Verfahren die folgenden Schritte umfasst:
- die Befestigung einer Klammer auf der Platte;
- die Bestimmung der Position und der Ausrichtung dieser Klammer im Verhältnis zur Plakette in Abhängigkeit von der Positionierung und der Ausrichtung der Vertiefungen und / oder der Spuren, die die Vertiefungen auf der Plakette trennen;
- die Abspeicherung:
o der Daten bezüglich der Positionierung und der Ausrichtung der Vertiefungen auf der Plakette,
o der Daten bezüglich der Positionierung und der Ausrichtung der Spuren, die die Vertiefungen auf der Plakette trennen, sowie
o der Daten bezüglich der Identifizierung der Plakette sowie der Daten bezüglich der Identität, der Position und der Ausrichtung der an der Plakette befestigten Klammer.

13. Verfahren für die gesicherte Ausgabe einer Einzeldosis eines Medikaments ausgehend von einer Plakette gemäß Anspruch 13, in der wenigstens ein Teil der abgespeicherten Daten auf der Klammer gespeichert ist.

14. Verfahren für die gesicherte Ausgabe einer Einzeldosis eines Medikaments ausgehend von einer Plakette gemäß Anspruch 13 oder 14, das umfasst:
- die Ausrichtung der Plakette im Verhältnis zu den Achsen des Messers, das zum Zuschneiden der Vertiefung der Plakette verwendet wird;
- das Zuschneiden der Vertiefung der Plakette.

15. Verfahren für die gesicherte Ausgabe einer Einzeldosis eines Medikaments ausgehend von einer Plakette gemäß Anspruch 13 bis 15, das die Befestigung der auf einer Karte zugeschnittenen Vertiefung umfasst, wobei diese Befestigung erst nach einer Bestätigung ausgeführt wird, dass über die Identität der zu befestigenden Vertiefung kein Zweifel besteht.

## Claims

1. Automatic system (1) for secure delivery of a unit dose of medicine starting from a blister pack comprising blisters containing the medicine, the system comprising:
- means of fixing a clamp on the blister pack;
- means of positioning and orienting this clamp relative to the blister pack as a function of the position and orientation of the blisters and/or grooves separating the blisters on the blister pack; and
- means of recording:
∘ data about the position and orientation of blisters on the blister pack,
∘ data related to the position and orientation of grooves separating the blisters on the blister pack, and
∘ data related to identification of the blister pack and data related to the identity, position and orientation of the clamp attached to the blister pack.

2. The automated system in claim 1 in which the clamp is oriented along the centre-line of one of the grooves separating the blisters and/or is positioned in two dimensions, the position in two dimensions being defined by an arrangement dependent on the geometric characteristics of the blister pack.

3. The automated system in claim 1 or 2, in which the clamp comprises means of recording data related to the position and/or orientation of blisters on the blister pack and/or data related to the position and orientation of grooves separating the blisters on the blister pack and/or data related to identification of the blister pack.

4. Automated system in claims 1 to 3, that also comprises:
- means of cutting out at least one blister in said blister pack, that are controlled by data related to the position and/or orientation of the blisters on the blister pack and/or by data related to identification of the blister pack, and/or
- that also comprises means of processing blisters taken from the blister pack that are controlled by data related to the position and/or orientation of blisters on the blister pack and/or by data related to identification of the blister pack.

5. Automated system according to claim 4, in which the blister pack and the cutting means are placed relative to each other depending on the data recorded in the clamp.

6. Automated system according to claims 1 to 5, provided with means of releasing the blister pack from the clamp.

7. Automated system according to one of claims 4 to 6, in which the means of processing blisters taken from the blister pack comprise means of maintaining the position and/or orientation of the blister during and after cutting.

8. Automated system according to one of claims 4 to 7, that also includes means of displacing the cutout blister while maintaining data about the position and/or orientation of the blister as cut out.

9. Automated system according to claim 8 that also comprises a gluing station and in which the displacement means put the blister into position in front of the gluing station during the path towards the attachment position on the board.

10. Automated system according to one of claims 1 to 9 that includes means of providing data to identify the medicine on the board after the blister containing the medicine has been cut out.

11. Automated system according to one of claims 1 to 10, comprising means of storing unit doses one by one, in the order in which they are produced in a case provided for this purpose.

12. Method for secure delivery of a unit dose of a medicine from a blister pack comprising at least one blister containing the medicine, the method including the following steps:
- attachment of a clamp onto the blister pack;
- determination of the position and orientation of this clamp relative to the blister pack as a function of the position and orientation of the blisters and/or grooves separating the blisters on the blister pack;
- recording of:
∘ data related to the position and orientation of the blisters on the blister pack;
∘ data related to position and orientation of the grooves separating the blisters on the blister pack; and
∘ data related to the identification of the blister pack and data related to the identity, position and orientation of the clamp attached to the blister pack.

13. Method for secure delivery of a unit dose of a medicine starting from a blister pack according to claim 13, in which at least part of the recorded data is recorded on the clamp.

14. Method for secure delivery of a unit dose of a medicine starting from a blister pack according to claim 13 or 14, that includes:
- orientation of the blister pack relative to the centre-lines of the blade used to cut out the blister in the blister pack;
- cutout of the blister in the blister pack.

15. Method for secure delivery of a unit dose of a medicine starting from a blister pack according to one of claims 13 to 15, that includes attachment of the cutout blister on a board, this attachment not being performed until after confirmation that there is no doubt about the identity of the blister to be attached.
